# EUROPEAN PATENT APPLICATION

(11) **EP 4 360 657 A1**
(43) Date of publication of application: **01.05.2024**
(21) Application number: 22383047.2
(22) Date of filing: 28.10.2022
(51) Int. Cl.: A61K 48/00, C12N 5/09, C12N 15/86, G01N 33/50

(54) **MIR-326 FOR THE TREATMENT OF CANCER**

(71) Applicant: Fundación para la Investigación Biomédica del Hospital Universitario Ramón y Cajal, 28034 Madrid (ES)
(72) Inventor: CONDE MORENO, Elisa, 28034 Madrid (ES); GARCÍA BERMEJO, María Laura, 28034 Madrid (ES); SERRANO HUERTAS, Silvia, 28034 Madrid (ES); CRESPO TORO, Lorena, 28034 Madrid (ES); RAMOS MUÑOZ, Miren Edurne, 28034 Madrid (ES); RODRÍGUEZ MARTÍN, Eulalia, 28034 Madrid (ES)
(74) Representative: Hoffmann Eitle

(57) **Abstract**

The present invention discloses a method of therapeutically treating cancer, in particular CRC (colorectal cancer), or of reducing the risk of cancer in particular CRC, disease progression in a patient comprising expressing miR-326 in the epithelial tumor cells and immune cells of said patient, preferably in NK and macrophages, and suitable means for said methods.

## Description

### Technical field

The present invention discloses a method of therapeutically treating cancer, in particular CRC (colorectal cancer), or of reducing the risk or the progression of cancer, in particular CRC disease progression, in a patient in need thereof, the method comprising expressing miR-326 in the epithelial tumor cells and/or immune cells of said patient, preferably in NK (Natural Killer cells) and macrophages, and suitable means for said methods.

### Background of the invention

A survival improvement in tumour patients and more particularly CRC patients has been observed in the past decades. This progress is largely attributed to the efforts and advances in screening and early detection but still, CRC (colorectal cancer) continues being the third leading cause of death in the world. Because of this, new effective biomarkers are currently being proposed to be valid and reliable for the diagnosis and prognosis of CRC disease and novel strategies are being developed for a more appropriate CRC patient management. The expression profile of microRNAs in the tissue of patients allows us to classify them into specific subgroups within the disease (Ronja S. Adam et al. 2022; Moreno et al. 2019). However, a low invasive method than a tissue biopsy is required for the diagnosis. In this sense, microRNAs can be found in different extracellular fluids including blood, serum, urine and saliva, among others, which allows an easy detection (Kim 2015). Moreover, miRNAs are highly stable molecules that avoid degradation by resisting room temperatures for several days and extreme conditions such as boiling, multiple freeze-thaw cycles, and high or low pHs (Huang 2017). These properties make their detection an easy and reproducible procedure worldwide. MicroRNA expression profile can be detected and monitored by qRT-PCR, a simple, cheap, reliable and well-established method to detect specific genes (Khoury et al. 2020). Furthermore, microRNAs are part of the pathophysiological mechanisms involved in the development and progression of the disease, which allow them to debut as early biomarkers that help to anticipate the diagnosis and the treatment of the tumour. In addition, most of the microRNAs are tissue-specific which lets ensuring a certain miRNA profile in plenty of patients suffering from a specific disease.

Due to all of this, miRNAs have been unleashed as novel targets for developing therapies against diseases (Huang 2017). To date, there are not microRNAs used in therapy to treat colorectal cancer, but there are some miRNAs modulators commercialised in other pathologies such as MiRavirsen, an intramuscular inhibitor of miR-122 for the treatment of Hepatitis C (Gebert et al. 2014), or ABX464, a molecule that triggers the increase of miR-124 to reduce the symptoms of inflammatory bowel disease for ulcerative colitis and Crohn's disease patients (Vermeire et al. 2021). Currently, some clinical trials are developing single miRNAs or microRNAs profile panels coming from the serum/urine of CRC patients to diagnose and predict CRC disease NCT02635087, NCT03362684, NCT04149613 www.clinicaltrials.gov.

Moreover, there are a multitude of companies working *in vivo* delivery vehicles for miRNA overexpression, or anti-miRNA constructs to successfully modulate miRNAs in animal models. The most extended methods used for *in vivo* miRNA modulation are based on viral particles, lentivirus (LV) or adeno-associated virus (AAV). Also, liposomes and polymers conjugated with the miRNA sequence are being validated as useful tools for this purpose (To et al. 2018). As recent gene therapies, new management needs to be carried out and a good equilibrium between the stability and the toxicity of these delivery vehicles is the main challenge of miRNA delivery (Haussecker 2014).

All this highlights the urgent clinical need of characterizing and validating new molecular targets for novel therapeutic approaches for CRC, including miRNAs as potential stable useful biomarkers of disease that can be detected in biological fluids. In this invention, we propose the use of miR-326 for novel therapeutic approaches for cancer, including CRC, including the use of this miRNA as a potential stable useful biomarker of disease, cancer disease and more particularly CRC, that can be detected in biological fluids.

In this sense, it has been widely described that miR-326 participates in essential processes of many types of tumours such as cellular apoptosis, tumour growth, cell invasion, embryonic development, immunomodulation, chemotherapy resistance, and oncogenesis (Y. Pan et al. 2019). Moreover, has been generally reported that this microRNA is downregulated in most cancers. In non-small lung cancer, miR-326 inhibits tumour proliferation by targeting CCND1 and high expression of the miRNA decreases tumour metastasis by targeting Adam17, NSBP1 and Phox2a (Cai et al., 2015; Sun et al., 2016; Wang et al., 2015). In gastric cancer, low expression of miR-326 is a prognostic factor for poor patient outcomes and downregulation of the miRNA promotes metastasis by targeting FSCN1 (Li et al. 2015). In breast cancer, miR-326 has been reported to repress cell metastasis by targeting B7-H3, a protein which correlates with adverse prognosis (Sun et al. 2014). miR-326 controls cancer invasiveness regulating VEGF-C and accelerates cervical cancer metastasis (Cheng et al. 2018; Zhang et al. 2017). The miRNA controls PKM2 which is overexpressed in thyroid cancer and plays an oncogenic role in metastasis (Yu et al., 2018). miR-326 targets the Bcl-2 axis inhibiting proliferation and promoting apoptosis in endometrial cancer (Cai et al. 2021). miR-326 expression level is restrained by abnormal PI3K signalling, resulting in a downregulation in glioblastoma (Nawaz et al. 2016).

The literature describes that miR-326 is acting in many types of cancer as a tumour suppressor miRNA, but little is known about the function of miR-326 in CRC. In the present invention, we gathered new evidence indicating that miR-326 modulation could be a potential therapeutic intervention to reduce or block tumour progression and could also be used as a prognostic biomarker in CRC.

### Brief description of the figures

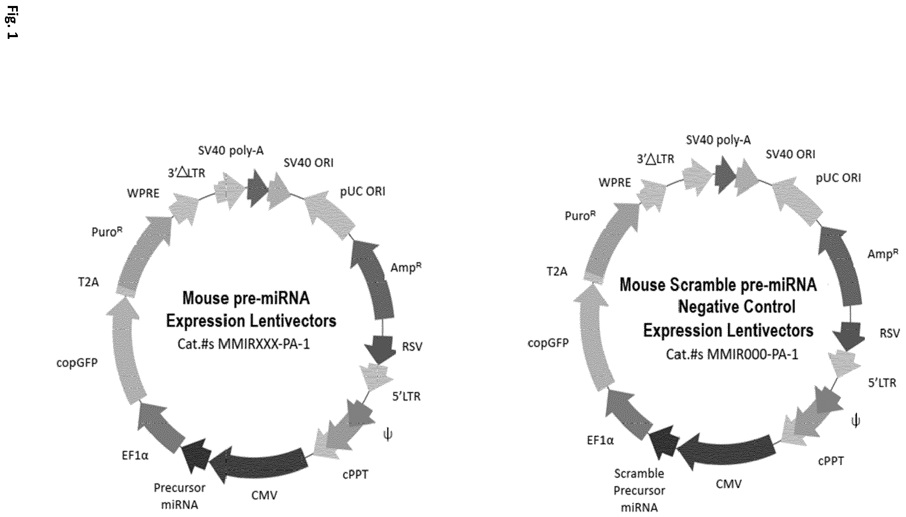

**Figure 1****.** Maps of lentiviral plasmids expressing the microRNA precursor and the microRNA Scrambled from SBI. The vectors contain common features: WPRE element enhances stability and translation of the CMV-driven transcripts. SV40 poly-A signal: enables efficient termination of transcription. Hybrid RSV-5'LTR promoter provides a high level of expression in producer 293 cells. Genetic elements (cPPT, GAG, LTRs): necessary for packaging, transducing, and stably integrating the viral expression construct into genomic DNA. SV40 origin: for stable propagation of the plasmid in mammalian cells. pUC origin: for high copy replication and maintenance of the plasmid in E. coli cells. Ampicillin resistance gene: for selection in E. coli cells. copGFP: fluorescent marker to monitor cells positive for transfection and transduction. Puromycin resistant gene: puromycin selection marker to enrich transuded cells and establish stable cell lines. The MMIR-326-PA-1 has 8872 bp and the MMIR-000-PA-1 has 8305 bp.

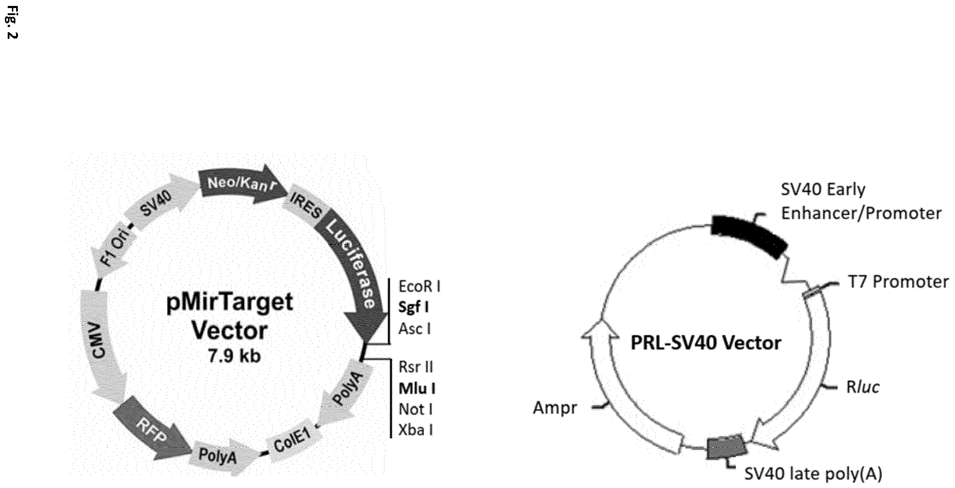

**Figure 2****.** Maps of pMirTarget Vector (Firefly Luciferase Vector) and pRL-SV40 Vector (Renilla Luciferase Normalization Vector). pMirTarget Vector includes the ITGA5 or CXCR4 sequence in the multiple cloning site downstream of luciferase stop codon. The presence of the appropriate miRNA causes a reduction of the luciferase activity. The vector contains: IRES-driven luciferase cassette, enables high sensitivity of Luciferase assay. RFP, reporter for transfection monitoring. Neomycin/Kanamycin, selection marker for stable cell establishment. Poly-A signal: enables efficient termination of transcription. SV40 origin: for stable propagation of the plasmid in mammalian cells. pRL-SV40 Vector contains common features: Antibiotic resistance (ampicillin), SV40 polyA and promoter and Luciferase gene. pMirTarget Vector contains around 8,000 bp and pRL-SV40 3500 bp Control vector. Abbreviations: Ampr, ampicillin resistance gene; Rluc, renilla luciferase gene; SV40 early enhancer/promoter, simian vacuolating virus 40 early enhancer/promoter.

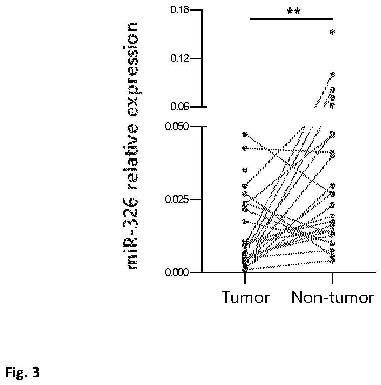

Figure 3. Analysis of miR-326 expression in tumour and adjacent non-tumour area of CRC patients by qRT-PCR. The graph shows that miR-326 expression is lower in tumour areas of CRC samples than in adjacent normal mucosa. miRNA expression levels were measured in 27 colorectal cancer biopsies. Data are shown as relative expression of miR-326 against normalizer. P-value showing statistical significance is indicated by **(P<0.01) following the paired samples Student's t-test.

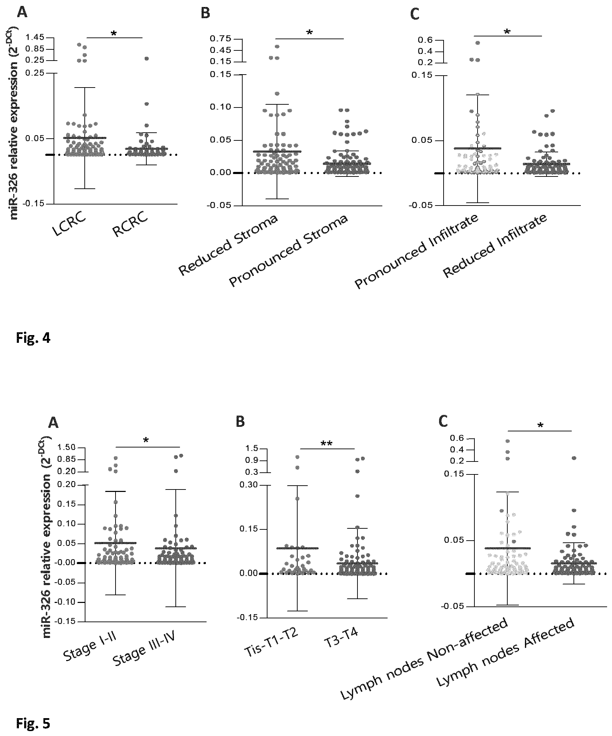

**Figure 4****.** miR-326 expression levels in CRC biopsies are associated with different histopathological features: tumour location, stroma percentage and immune infiltrate. miR-326 expression levels measured in 192 paraffin-embedded tumour biopsies statistically associated with: (A) tumor location: miR-326 expression is higher in LCRC (left-sided colorectal cancer) than RCRC (right-sided colorectal cancer) (B) tumor stroma abundance: high miR-326 expression is associated with reduced stroma (C) immune infiltrate: high miR-326 expression correlates with pronounced infiltrate. miR-326 levels are expressed as 2-ΔCt. Statistical analysis was performed using SPSS 19.0. P-value showing statistical significance is indicated by ^{∗}(P<0.05).
**Figure 5****.** miR-326 expression levels in CRC biopsies are associated with different histopathological features: tumor staging and invasion. miR-326 expression levels measured in 192 paraffin-embedded tumour biopsies are statistically associated with: (A) TNM stages: miR-326 expression is higher in low TNM (I-II) than in high TNM stages (III-IV) (B) tumor invasiveness grade (T1-T4): high miR-326 expression is associated with early CRC stages compared to late stages (T3-T4) (C) lymph nodes affectation: miR-326 expression is lower when lymph nodes are affected compared with lymph nodes not-affected. miR-326 levels are expressed as 2-ΔCt. Statistical analysis was performed using SPSS 19.0. P-value showing statistical significance is indicated by ^{∗}(P<0.05) and ^{∗∗}(P<0.01).

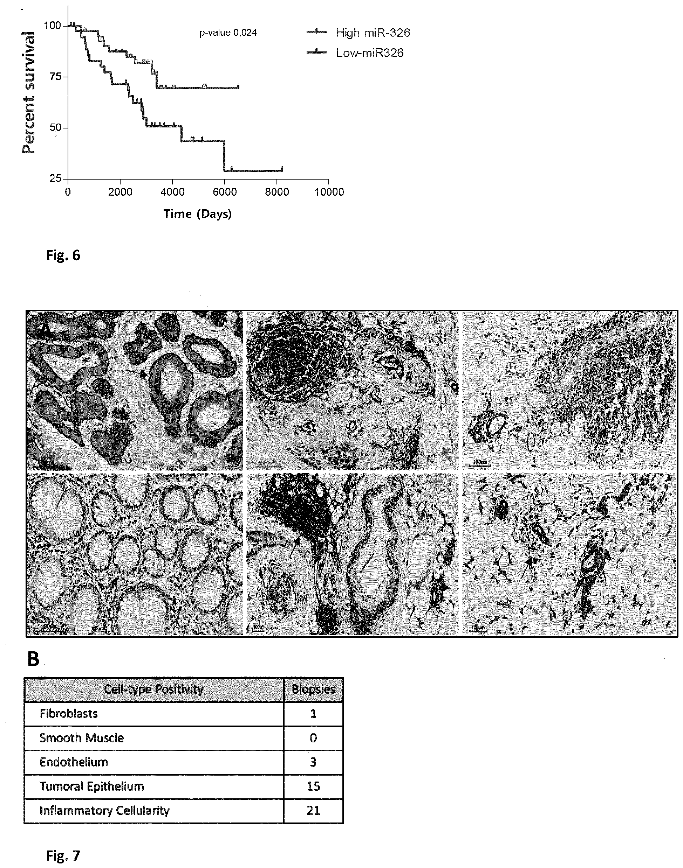

**Figure 6****.** miR-326 expression levels are associated with overall survival in CRC patients. The graph shows that miR-326 expression levels are statistically associated with the overall survival of patients (days). High expression of miR-326 is significantly associated with longer overall survival compared to low levels of the miRNA. miRNA expression levels were measured in 86 tumour biopsies. P-value (0,024) showing statistical significance is indicated in the graph.
**Figure 7****.** Localization and quantification of miR-326 in different cell compartments within CRC biopsies. (A) miR-326 location was evaluated by in situ hybridization in 40 biopsies of CRC patients by a specialized pathologist. miR-326 is localized in different compartments such as epithelial tumour cells, fibroblasts, inflammatory cells and endothelium. miR-326 signalling is visualized in purple. (B) Quantification of the number of biopsies in which miR-326 expression is located. miR-326 is mainly localised in tumour epithelial and inflammatory cells.

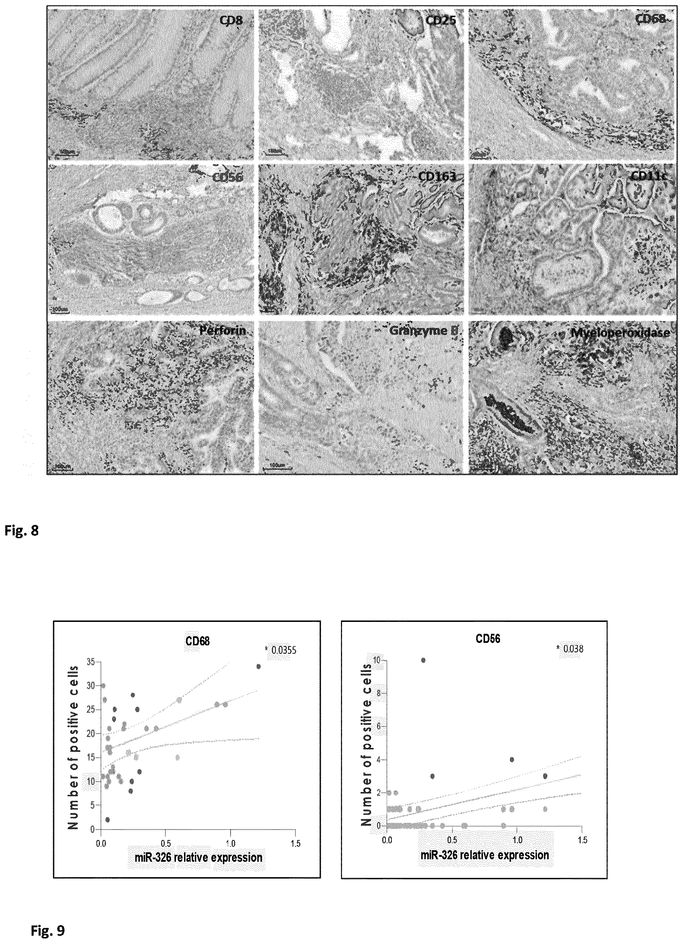

**Figure 8****.** Characterization of the inflammatory infiltrate in tumour biopsies of CRC patients by immunohistochemistry. Representative images of CD8, CD25, CD68, CD56, CD163, CD11c, Perforin, Granzyme B and Myeloperoxidase immunostainings are shown. CD8 is a marker for cytotoxic T cells, CD25 binds to T cells and mastocytes, CD68 marks monocytes and M1 macrophages, CD56 reveals NK cells, CD163 binds to M2 macrophages. CD11c marks dendritic cells, Perforin and Granzyme B recognise cytotoxic lymphocytes CTL and NK cells and Myeloperoxidase detects granulocytes and mastocytes. The signalling is visualized in brown (zoom: x10).
**Figure 9****.** Correlation between miR-326 expression by qRT-PCR and CD68 and CD56 positive cells by immunohistochemistry. A positive correlation was found between miR-326 expression levels and CD68, M1 macrophage marker and CD56, NK cells marker. These data were measured in 40 paraffin-embedded tumour biopsies. Statistical differences between miR-326 levels and these immune cells markers are expressed as 2-ΔCt using the mean of miR-103 and miR-191 Cq values as normalizer. P-value showing statistical significance is indicated by ^{∗}(P<0.05).

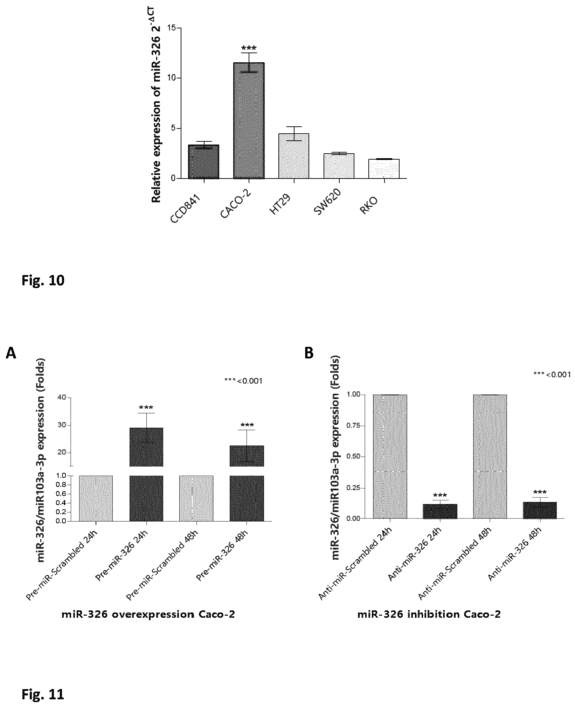

**Figure 10****.** miR-326 basal expression in tumoral and non-tumoral colorectal cells. miR-326 basal levels were measured in Caco-2, HT-29, SW620, RKO and CCD841 cells by qRT-PCR amplification. Statistical significance of miR-326 expression levels was found in Caco-2 cells compared to the rest of the cell lines. HT-29, SW620 and RKO cells do not present statistically significant differences. These levels are expressed in relative expression of miR-326 calculated by formula 2(-ΔCt) and using hsa-miR-103a-3p as normalizer. Data are presented as mean ± SD of nine independent experiments. Asterisks indicate statistical significance ***(P<0.0001).
**Figure 11****.** miR-326 modulation in Caco-2 cells by transient transfection. miR-326 expression levels after miRNA modulation expressed as fold change values using hsa-miR-103a-3p as normalizer. Transient transfection was carried out at 24h and 48h. (A) Overexpression and (B) inhibition of miR-326 have been successfully achieved. miR-326 expression levels were obtained by qRT-PCR amplification, calculated by formula 2(-ΔΔCt) comparing pre-miR with its control pre-miR-Scrambled and Anti-miR with Anti-miR-Scrambled. Data are presented as mean ± SD of seven independent experiments. Asterisks indicate statistical significance by Mann Whitney Test ***(P<0.001).

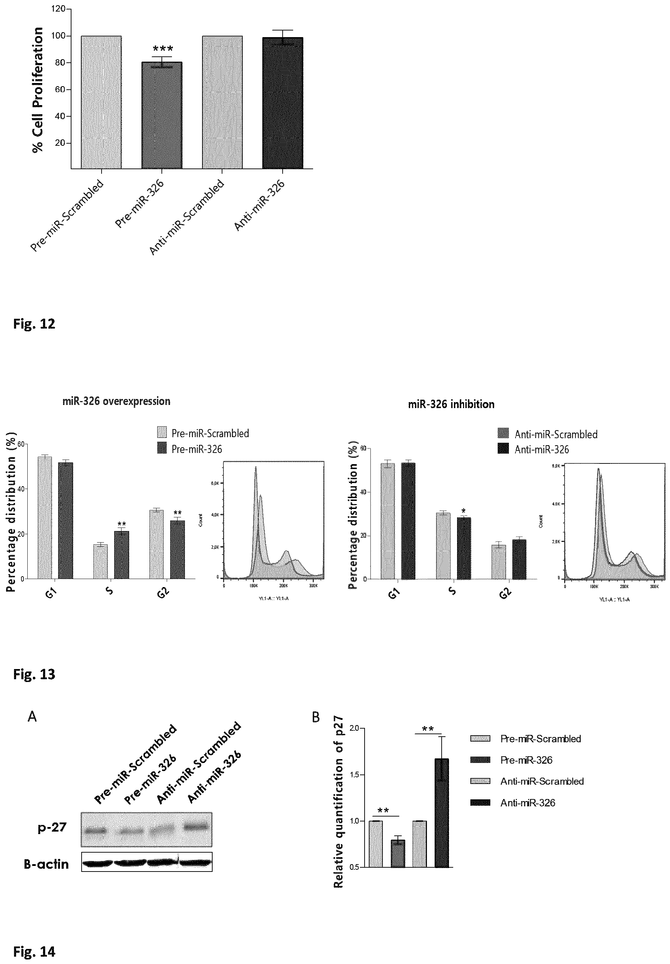

**Figure 12****.** Cell proliferation assay in miR-326 modulated Caco-2 cells by BrdU analysis. Overexpression of miR-326 in Caco-2 cells shows a decrease in the proliferation rate. Inhibition of miR-326 did not show differences in cell proliferation process. Data are represented as percentage of proliferation process with BrdU staining as mean ± SD of six independent experiments. Asterisks indicate statistical significance ***(P<0.001).
**Figure 13****.** Percentage distribution of cell cycle phases in miR-326 modulated Caco-2 cells. Modulation of miR-326 was performed for 72 hours in Caco-2 cells. Cell cycle analysis shows that (A) overexpression of miR-326 arrests cells in S-phase decreasing the number of cells in G2 phase. (B) Inhibition of miR-326 decreased the number of cells in S-phase. Analysis of the cell cycle in G0/G1, S and G2/M phases was determined using the FlowJOv10 program. Data were represented as percentage distribution of cells in each cell cycle phase as mean ± SD of eleven independent experiments. Asterisks indicate statistical significance *(P<0.05), **(P<0.01).
**Figure 14****.** Analysis of p27 expression in miR-326 modulated Caco-2 cells by immunoblot. (A) Representative image of p27 protein levels estimated by western blot using β-actin as a loading control. P27 protein expression decreases in pre-miR-326 conditions and increases in Anti-miR-326, comparing each condition with its Scrambled. (B) Quantification of mean ± SD of seven independent experiments of miR-326 modulated Caco-2 cells. Asterisks indicate statistical difference ** (P<0.01).

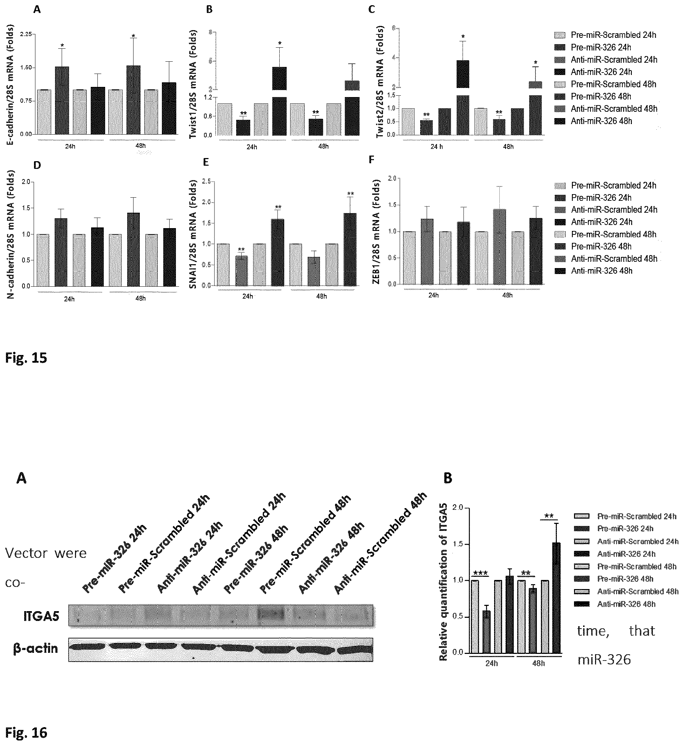

**Figure 15****.** Analysis of EMT genes expression levels in miR-326 modulated Caco-2 cells by qRT-PCR. Expression from epithelial (E-cadherin) and mesenchymal genes (TWIST1, TWIST2, N-cadherin, SNAI1, and ZEB1) were determined by qRT-PCR in Caco-2 cells at 24-h and 48-h post-miR-326 modulation. (A) E-cadherin expression increases in miR-326 overexpressed Caco-2 cells compared with scrambled group. (B, C, E) TWIST1, TWIST2 and SNAI1 expression was reduced in the Pre-miR-326 condition and increased in the Anti-miR-326 group compared with Pre-miR-Scrambled and Anti-miR-Scrambled, respectively. (D, F) N-cadherin and ZEB1 did not show differences with miR-326 modulation. 28S ribosomal gene was used to normalize genes expression. Asterisks indicate statistical significance ^{∗}(P<0.05), ^{∗∗}(P<0.01). Data are presented as mean ± SD of six independent experiments, as fold change values.
**Figure 16****.** ITGA5 expression in miR-326 modulated Caco-2 cells determined by Immunoblot assays. (A) Representative image of ITGA5 protein levels estimated by western blot using β-actin as a loading control. ITGA5 protein expression decreases in pre-miR-326 conditions and increases in Anti-miR-326, comparing each condition with its Scrambled. (B) Quantification of seven different immunoblot assays of miR-326 modulated Caco-2 cells. Asterisks indicate statistical difference ** (P<0.01) and ***(P<0.001).

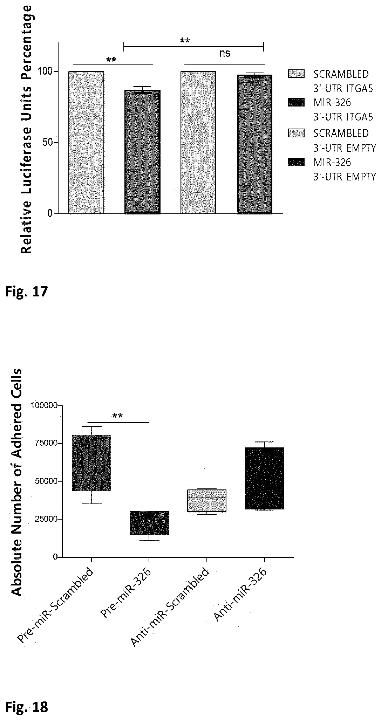

**Figure 17****.** ITGA5 Luciferase assay in miR-326 modulated Caco-2 cells. Luciferase assays of 3'-UTR ITGA5 vectors and 3'-UTR Empty vectors were carried out in miR-326 modulated Caco-2 cells. A decrease in luciferase activity was observed between Pre-miR-Scrambled and Pre-miR-326 transfected cells **(P<0.01). No statistical significance was observed between Pre-miR-326 and Pre-miR-Scrambled 3'-UTR Empty Vector. Luciferase activity percentage is represented using renilla activity as normalization control. Data are presented as mean ± SD of six experiments.
**Figure 18****.** Cell adhesion assay to fibronectin matrix in miR-326 modulated Caco-2 cells. Overexpression of miR-326 in Caco-2 cells shows a decrease of cells attaching to the fibronectin matrix. No statistical significance was observed when miR-326 was inhibited. Data are presented as absolute number of adhered cells as mean ± SD of five independent experiments. Asterisks indicate statistical significance **(P<0.01).

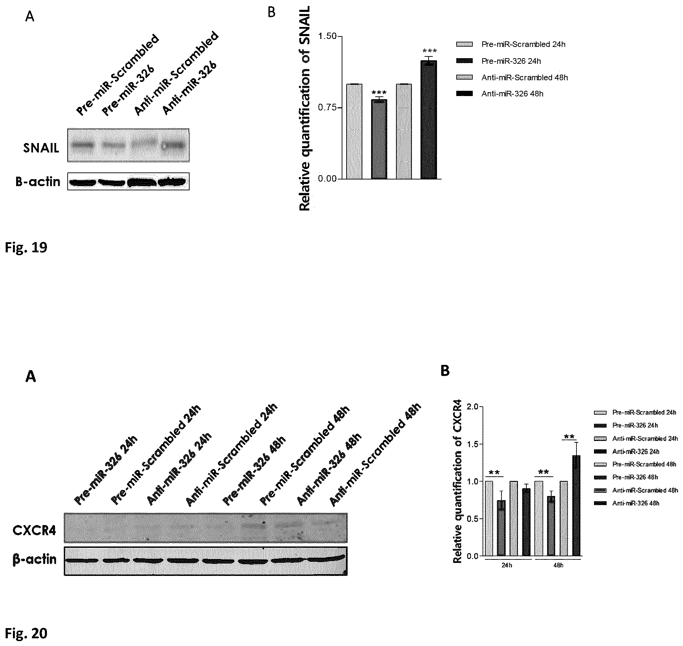

**Figure 19****.** Analysis of SNAIL expression in miR-326 modulated Caco-2 cells by immunoblot. (A) Representative image of Snail protein levels estimated by western blot using β-actin as a loading control. Snail protein expression decreases in pre-miR-326 conditions and increases in Anti-miR-326, comparing each condition with its Scrambled. (B) Quantification of seven different immunoblot assays of miR-326 modulated Caco-2 cells. Asterisks indicate statistical difference ** (P<0.01).
**Figure 20****.** CXCR4 expression in miR-326 modulated Caco-2 cells by Immunoblot assays. (A) Representative image of CXCR4 protein levels estimated by western blot using β-actin as a loading control in miR-326 modulated Caco-2 cells at 24h and 48-hours. CXCR4 expression decreases in Pre-miR-326 conditions at 24h and 48h and increases in Anti-miR-326 at 48h, comparing each condition with its corresponding Scrambled. (B) The graphic shows seven different immunoblot assays quantification. Asterisks indicate statistical significance comparing each sample with its Scrambled ^{∗∗}(P≤0.01).

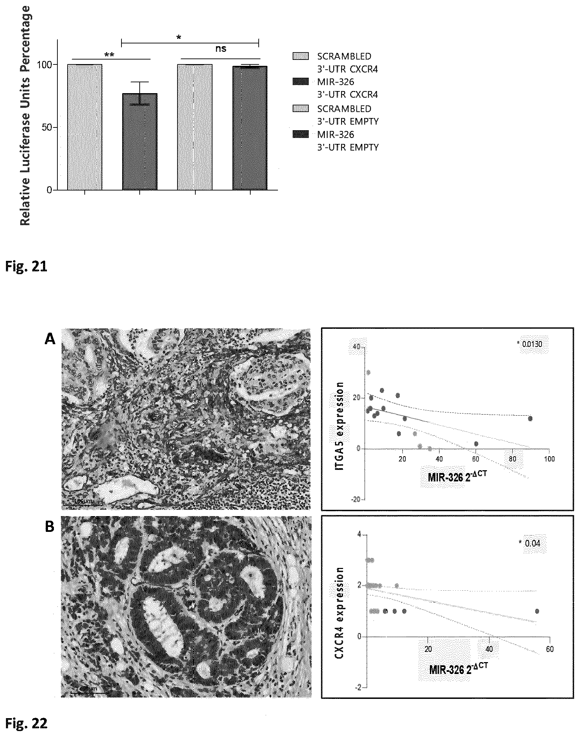

**Figure 21****.** CXCR4 Luciferase assay in miR-326 modulated Caco-2 cells. Luciferase assays of 3'-UTR CXCR4 vectors and 3'-UTR Empty vectors were carried out. A decrease in luciferase activity is observed between Pre-miR-Scrambled and Pre-miR-326 transfected cells in the 3'-UTR CXCR4 vectors **(P<0.01). No statistical significance is observed between Pre-miR-326 and Pre-miR-Scrambled in the 3'-UTR Empty Vectors. Luciferase activity percentage is represented, using renilla activity as normalization control. Data are presented as mean ± SD of six experiments.
**Figure 22****.** Immunostaining and correlation study of ITGA5 and CXCR4 to characterize the potential miR-326 targets in CRC biopsies. (A) A representative image of 40 ITGA5 stained tissues is shown. ITGA5 expression was quantified, and inverse correlation with miR-326 expression was found. (B) A representative image of 40 CXCR4 stained tissues is shown. CXCR4 expression was quantified, and inverse correlation with miR-326 was found. CXCR4 was quantified as a CXCR4 Score, combining the expression extension and intensity in epithelial tumor cells. miR-326 expression levels were determined by qRT-PCR amplification, calculated by formula 2(-ΔCt). Asterisks indicate statistical significance ^{∗}(P<0.05).

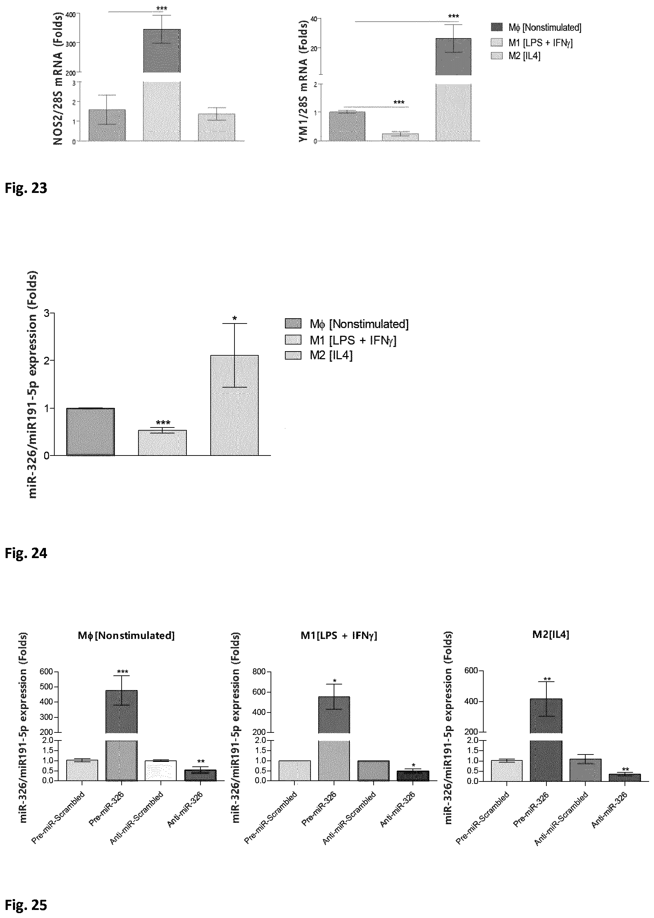

**Figure 23****.** Macrophage polarization markers in different induced phenotypes (M1[LPS + IFNy] and M2[IL-4]) compared to naive macrophages MΦ[Non-stimulated] by qRT-PCR. Graphics show induced expression levels of NOS2 in the proinflammatory M1[LPS + IFNy] phenotype in comparison with the MΦ[Non-stimulated] macrophages. Conversely, YM1 shows higher expression in the M2[IL-4] phenotype compared to both other experimental groups. 28S ribosomal gene was amplified and used to normalize M1 and M2 genes expression. Data from qRT-PCR are presented as mean ± SD independent experiments M1[LPS + IFNy] N=10, M2[IL-4] N=7 and MΦ[Non-stimulated] N=7. Significant differences between experimental groups are indicated, as fold change values by ***(P<0.001).
**Figure 24****.** miR-326 expression in different induced phenotypes (M1[LPS + IFNy] and M2[IL-4]) compared to naive macrophages Mφ[Non-stimulated] by qRT-PCR. Different miR-326 expression levels were found in each macrophage stimulation group. Low levels of miR-326 were found in M1[LPS + IFNy] and high levels of miR-326 in M2[IL4] compared to non-stimulated macrophages, as control group. hsa-miR-191-5p was used to normalize miR-326 expression. Data are presented as mean ± SD of twelve independent experiments. Significant differences with, are indicated by ^{∗}(P<0.05) ^{∗∗∗}(P<0.001).
**Figure 25****.** Efficiency of miR-326 modulation in naive macrophages MΦ[Non-stimulated] and different induced phenotypes (M1[LPS + IFNy] and M2[IL-4]) by transient transfection. miR-326 overexpression and inhibition were successfully achieved compared to Pre-miR-Scrambled and Anti-miR-Scrambled as control groups in each stimulation. hsa-miR-191-5p was used to normalize miR-326 expression. Data are presented as mean ± SD of six independent experiments. Significant differences with, are indicated by ^{∗}(P<0.05) ^{∗∗}(P<0.01) as fold change values.

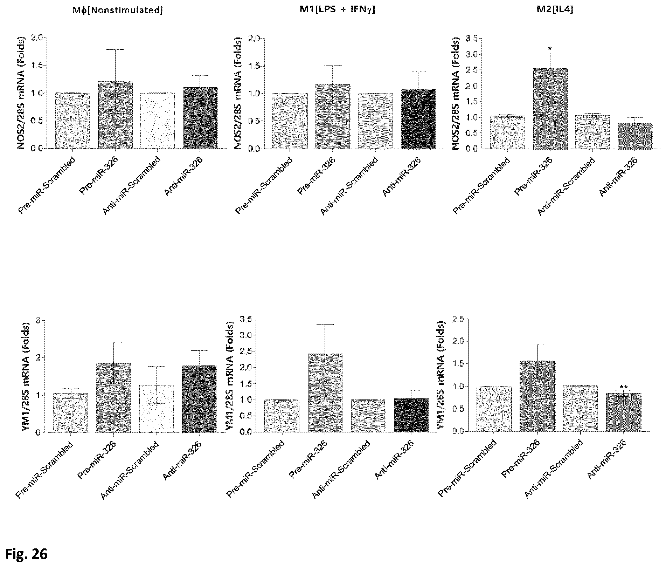

**Figure 26****.** Macrophage phenotype markers analysis in naive macrophages Mφ[Non-stimulated] and induced phenotypes (M1[LPS + IFNy] and M2[IL-4]) after miR-326 modulation by transient transfection. MΦ[Non-stimulated] and M1[LPS + IFNy] macrophages do not show differences in NOS2 and YM1 gene expression after miR-326 overexpression or inhibition. miR-326 overexpression in M2[IL4] stimulated macrophages lead to a significant induction of NOS2 expression. On the contrary, YM1 expression decreased when miR-326 was inhibited in M2[IL4] stimulated macrophages Data are presented as mean ± SD of six independent experiments. Significant differences with, are indicated by ^{∗}(P<0.05) ^{∗∗}(P<0.01) as fold change values.

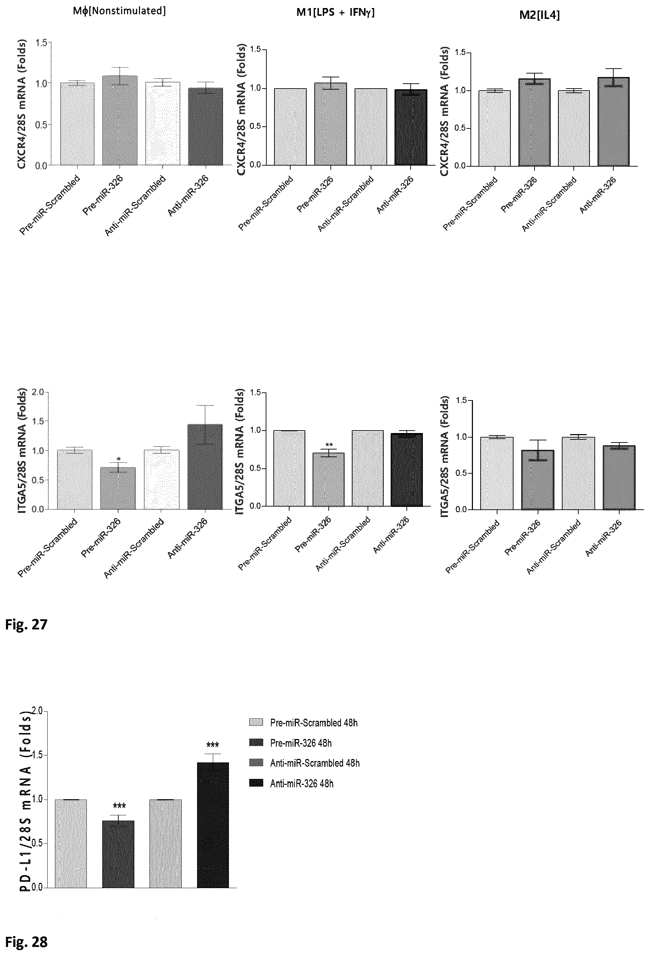

**Figure 27****.** Analysis of CXCR4 and ITGA5 expression in miR-326 modulated Mφ[Non-stimulated], M1[LPS + IFNy] and M2[IL-4] macrophages by qRT-PCR. Graphics do not show differences of CXCR4 expression in miR-326 modulated macrophages. ITGA5 expression decreases in Mφ[Non-stimulated], M1[LPS + IFNy] macrophages overexpressing miR-326. Inhibition of miR-326 does not affect ITGA5 expression. Data are presented as mean ± SD of eight independent experiments. Significant differences with, are indicated by ^{∗}(P<0.05) ^{∗∗}(P<0.01) as fold change values.
**Figure 28****.** PD-L1 gene expression in miR-326 modulated Caco-2 cells by qRT-PCR. Graphic shows differences of PD-L1 expression measured by qRT-PCR in miR-326 modulated Caco-2 cells at 48h post-transfection. PD-L1 expression decreases in Caco-2 cells overexpressing miR-326 and increases when miR-326 is inhibited compared to each Scrambled condition. Data are presented as mean ± SD of eight independent experiments. Significant differences with, are indicated by ***(P<0.001) as fold change values.

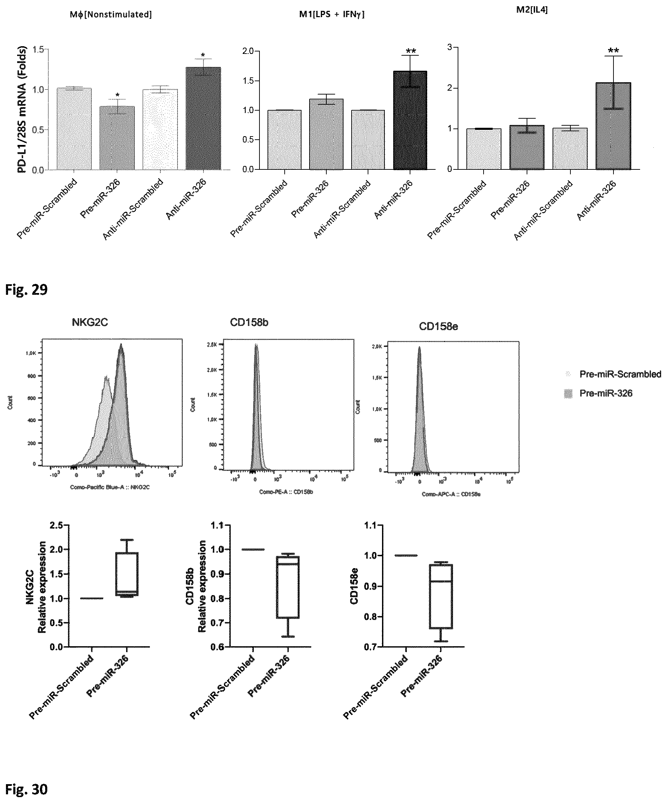

**Figure 29****.** PD-L1 gene expression in modulated Mφ[Non-stimulated], M1[LPS + IFNy] and M2[IL-4] macrophages by qRT-PCR. Graphics show differences in PD-L1 expression in miR-326 modulated macrophages. PD-L1 expression decreases in MΦ[Non-stimulated] macrophages overexpressing miR-326 and increases when miR-326 is inhibited. PD-L1 expression in M1[LPS + IFNy] and M2[IL-4] increases with miR-326 inhibited macrophages. Overexpression of the miRNA in these conditions does not show differences. Data are presented as mean ± SD of ten independent experiments. Significant differences with, are indicated by ^{∗}(P<0.05) ^{∗∗}(P<0.01) as fold change values.
**Figure 30****.** Cytotoxicity regulators expression study in miR-326 modulated NK92 cells by flow cytometry. miR-326 overexpression in NK92 cells increases the expression of the cytotoxicity activation marker, NKG2C. Moreover, miR-326 overexpression in NK92 cells decreases the expression of the cytotoxicity inhibition markers CD158b and CD158e. Data are presented as mean ± SD of 3 independent experiments.

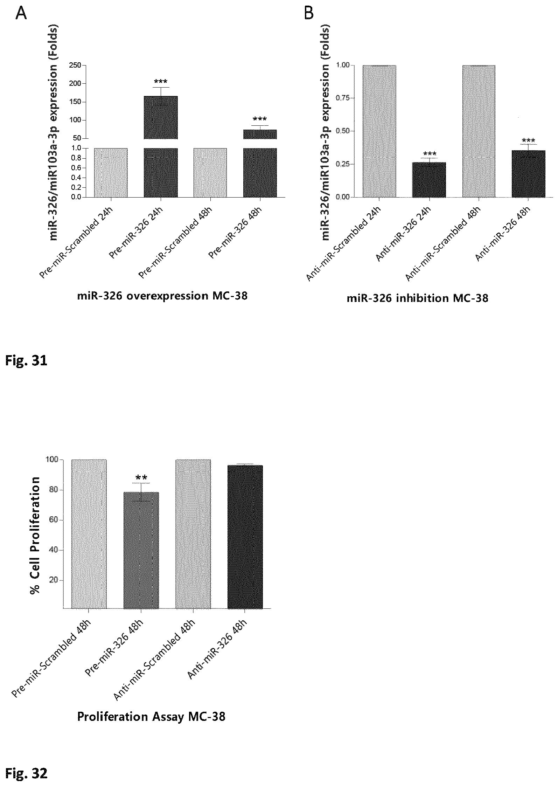

**Figure 31****.** miR-326 modulation in MC-38 cells by transient transfection. miR-326 expression levels after miRNA modulation expressed as fold change values using hsa-miR-103a-3p as housekeeping. Transient transfection was carried out at 24h and 48h. (A) Overexpression and (B) inhibition of miR-326 have been successfully achieved. miR-326 expression levels were obtained by qRT-PCR amplification, calculated by formula 2(-ΔΔCt) comparing pre-miR with its control pre-miR-Scrambled and Anti-miR with Anti-miR-Scrambled. Data are presented as mean ± SD of seven independent experiments. Asterisks indicate statistical significance by Mann Whitney Test ***(P<0.001).
**Figure 32****.** Cell proliferation analysis in miR-326 modulated MC-38 cells. Cell proliferation analysis through BrdU assays demonstrates that overexpression of miR-326 leads to a decrease in proliferation rate. Inhibition of miR-326 did not show differences in cell proliferation process. Modulation of miR-326 was performed for 72 hours in MC-38 cells. Data were represented as percentage of cells with BrdU staining as mean ± SD of five independent experiments. Asterisks indicate statistical significance by Mann Whitney Test **(P<0.01).

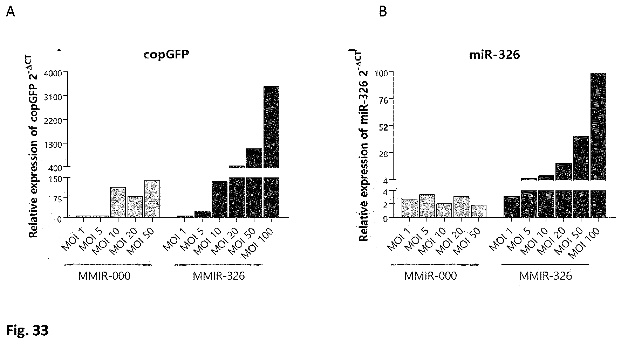

**Figure 33****.** CopGFP and miR-326 expression in MMIR-326 LV MC-38 modulated cells. A gradient of MOls was carried out from MOI 1, 5, 20, 50 for miR-Scrambled and MOI 1, 5, 10, 20, 50 and 100 for miR-326. (A) Efficient infection of the virus is achieved in MMIR-326 but not in MMIR-000 determined by CoGFP amplification. (B) miR-326 modulation is properly achieved determined by miR-326 amplification. Expression data were obtained by qRT-PCR amplification, calculated by formula 2(-ΔΔCt) comparing MMIR-326 with its control MMIR-000. Data are presented as mean ± SD of two independent experiments.

### Description of embodiments

In this invention, we have identified miR-326 as a key mediator involved in the development and progression of colorectal cancer unveiling this molecule as a biomarker and therapeutic target in this tumoral context.

We have identified hsa-miR-326 as a potential biomarker of CRC due to its expression associated with different histopathological features like tumour appearance, tumour location, stroma percentage, immune infiltrate, tumour staging and invasion in a cohort of 192 CRC patients. Moreover, we demonstrated that high levels of miR-326 are associated with a better patient's overall survival. Furthermore, we have determined that miR-326 is mostly located in tumour epithelial cells, macrophages, and natural killer cells in colorectal cancer patients' biopsies. Moreover, we have induced and inhibited miR-326 expression in Caco-2, an epithelial colorectal cancer cell line, and in Mφ, M1 and M2 murine macrophages. We demonstrated that the overexpression of the microRNA in Caco-2 cells decreases cell proliferation arresting the colon cancer cells in S-phase and reduces expression of mesenchymal-transition related genes as TWIST1/2 and SNAIL. Furthermore, we have identified ITGA5, as a direct target gene of miR-326 and we demonstrated that this miRNA attenuates cell adhesion to a malignant fibronectin matrix, being this protein the principal receptor of ITGA5. Moreover, we have identified another direct target of the miRNA in Caco-2 cells, CXCR4. In addition, we have identified an inverse correlation of miR-326 expression with these two targets in patients' biopsies. In an inflammatory context, we have demonstrated *in vitro* that different miR-326 expression levels are found in Mφ, M1 and M2 murine macrophages polarized upon different stimuli. Modulation of miR-326 by transient transfection does not regulate macrophage polarization but is involved in the process that leads macrophages to a M1 phenotype. Additionally, we suggest ITGA5 as a potential target of miR-326 in Mφ and M1 macrophages. Finally, we have demonstrated that overexpression of miR-326 reduces PD-L1 levels in Caco-2 cells, Mφ, M1 and M2 murine macrophages and inhibition of the miRNA leads to the opposite effect, PD-L1 levels increased. Moreover, overexpression of miR-326 in NK cells increases the expression of the cytotoxicity activation marker, NKG2C and reduces the expression of the cytotoxicity inhibition markers CD158b and CD158e. All these results led us to establish a suitable experimental mice model to study the role of miR-326 during CRC progression by subcutaneous injection of MC-38 murine cells upon miR-326 modulation. We have transiently modulated miR-326 expression in MC-38 cells, demonstrating that its overexpression reduces proliferation in this murine cell type. We have established miR-326 overexpression in MC-38 cells through lentiviral vectors.

On the other hand, the present invention is the first one that analyses miR-326 expression in a large cohort of CRC patients with 192 biopsies from primary tumours and relates the microRNA with tumour-infiltrating immune cells and overall survival.

We characterized the differences in the expression pattern of miR-326 in colorectal cancer biopsies compared to healthy patients' tissues. In our cohort, the expression levels of miR-326 were decreased in colorectal tumour sections compared to their corresponding adjacent mucosa tissues. Taking this into account, we herein indicate that miR-326 expression is an early diagnostic biomarker of colorectal cancer.

Furthermore, in our patients' cohort, miR-326 was upregulated in left-side tumours compared to right-side tumours. The distinction between right- and left-sided colon is based on the embryological origin and both sides exhibit different histology. Since LCRC is clearer to identify in the early stages of carcinogenesis because presents polypoid morphology, RCRC exhibits flat morphology that is difficult to detect, presenting a more aggressive phenotype (Baran et al. 2018; Mukund et al. 2020). This sidespecificity exhibits therapy responses completely different, LCRC patients benefit more from chemotherapies treatments than RCRC which have demonstrated promising results with immunotherapy. Moreover, the side-distinction presents post-transcriptional regulation miRNAs differences. All of this points out that miR-326 associates with the less aggressive phenotype and suggest that miR-326 could be used as a predictive biomarker with reference to the different treatments employed for CRC patients.

Furthermore, we demonstrated that miR-326 expression was associated with tumour stroma percentage. Our results show that high levels of miR-326 were linked to reduced stroma patients and correlate with longer overall survival. The stroma is a critical component of the tumour microenvironment and possesses certain tumour-suppressing abilities. Despite this, tumour malignancy changes the stroma profile and tumour-associated stromal cells contribute to local and distant progression of the malignant tumour (Bremnes et al., 2011; Valkenburg et al., 2018). Tumours with a high stroma percentage exhibited poor patient prognosis in CRC patients (Park et al. 2014). All this suggests that this miRNA could be able to predict the prognosis of the patients with the differential expression of miR-326.

In addition, miR-326 presented a strong correlation with immune infiltrate profile in our patient's cohort showing that high levels of miR-326 correlated with high tumour infiltrating immune cells. This suggests that miR-326 could be established as a good prognostic biomarker in terms of immune infiltrate profile of CRC patients.

All these findings indicate for the first time that increased miR-326 levels associated with left-sided tumours, reduced stroma and pronounced infiltrate which link with a better prognosis of CRC patients.

Furthermore, miR-326 was associated with tumour stage and invasion in our patients' cohort. Increased levels of miR-326 were detected in earlier stages of the disease (TNM I-II) compared to late stages (TNM III-IV) of CRC patients. Consistently to the tumour stage, miR-326 presented a correlation with tumour invasiveness grade (T1-T4). miR-326 was upregulated in our patients' cohort when the tumour had not been extremely invasive (Tis-T1-T2) compared to late stages of invasiveness (T3-T4). Moreover, miR-326 was also related to the lymph nodes affectation. In our cohort, miR-326 levels were upregulated when lymph nodes had not been affected compared to lymph-node's affectation.

The differential of miR-326 expression along the progression of the disease shows that this miRNA could be an efficient prognostic biomarker for CRC patients. Our results have demonstrated for the first time in patients that low miR-326 levels are associated with tumour staging, invasion and metastasis, which correlates with a worse prognosis for CRC patients.

Related to that, we described that miR-326 expression levels were associated with patient's overall survival. High levels of the microRNA were associated with better patients' overall survival. Therefore, again, our results demonstrated for the first time that miR-326 acted as a potential prognostic biomarker in CRC patients and could be considered a potential therapeutic target for colorectal cancer.

On the other hand, the malignant phenotypes of the different types of cancers are defined not only by the intrinsic activities of tumour cells but also by the immune cells recruited in the tumour-related microenvironment (Ben-Baruch 2002), and the interaction between tumour and immune cells has significant prognostic relevance in tumour progression (Bremnes et al., 2011). Therefore, in recent years, researchers have focused their investigation on the role of miRNAs in inflammatory and anti-inflammatory processes. Many studies have confirmed that miRNAs dysregulation in cells and targeted tissues is correlated with immune disequilibrium and autoimmune diseases (Jadideslam et al. 2018). Recent research has described miR-326 is critical in regulating functions of inflammatory cells mostly in the pathogenesis of neurodegenerative and autoimmune disorders. miR-326 coordinates TH-17 differentiation and correlates with leukocytes in multiple sclerosis (Du et al. 2009; Honardoost et al. 2014), plays a role in autoimmune thyroiditis regulating Th17/Treg balance (Zhao et al. 2021) or contributes to B cell hyperactivity in systemic lupus erythematosus (Xia et al. 2018). We analysed which kind of immune cells presented a correlation with miR-326 expression in our patients' cohort. A significant positive correlation was found between miR-326 levels and CD68, the classical M1 macrophage marker, and CD56, the classical marker of natural killer cells. We demonstrated that high levels of the microRNA were related to increased levels of macrophages and NK cells. Furthermore, no correlation was found between miR-326 expression and CD163, the classical marker of M2 macrophages. In connection with our results, Li et al., published in 2017 that miR-326 was involved in macrophage activation in liver injury and increased levels of miR-326 were correlated with low levels of M1 macrophage phenotype. Furthermore, Bai et al., published in 2020 that exosomes containing miR-326 were released by M1 macrophages to suppress proliferation, migration and invasion in hepatocellular carcinoma. These dates suggest the miR-326/macrophage interaction can be an important regulator pathway in different types of diseases, but no one has reported this association in CRC. Furthermore, no correlation was found in the bibliography between miR-326 and natural killer cells, but it has been reported that NK cells decrease with CRC TNM-stage increase (Guo et al. 2020). Therefore, our results have demonstrated for the first time that miR-326 correlates positively with M1 macrophages and NK cells in colorectal cancer patients.

To study the implication of miR-326 in CRC development and progression-related processes, we have set up the exogenous modulation of miR-326 in Caco-2 cells. We demonstrated that the overexpression of the miRNA decreases the proliferation in this colorectal cancer cell line in concordance with the bibliography. It has been previously described that miR-326 overexpression inhibits *in vitro* cell proliferation and suppresses *in vivo* tumour growth in different kinds of tumours such as melanoma (Kang et al. 2017), endometrial cancer (Liu et al., 2017), cervical cancer (Zhang et al. 2017), breast cancer (Ghaemi et al. 2019), prostatic carcinoma (Liang et al., 2018) or lung cancer (Sun et al., 2016; Wang et al., 2016). Furthermore, miR-326 has been already reported to reduce cell proliferation in SW480 colorectal cancer cells by inhibiting E2F1, a member of the E2F gene family involved in cell cycle regulation (Gao et al., 2021). However, the decrease of proliferation process overexpressing miR-326 had not been described to date in Caco-2 cells.

Moreover, we studied the cell cycle distribution after modulating miR-326 in Caco-2. The overexpression of the miRNA showed a smaller number of cells entering in G2/M phase. This effect is produced because Caco-2 cells are arrested in the S-phase and cannot continue progressing through the cell cycle phases. The opposite effect was achieved by inhibiting miR-326, and a significant reduction in the percentage of S-phase cells was found and a bigger number of cells were entering in mitosis. This effect may be due, among other things, to a deregulation in some cell cycle regulators. In many tumours, regulators of the cycle are altered, and uncontrolled expression/activity of these proteins shows a deregulated cell cycle control. As it was described in the results section, overexpression of miR-326 reduces p27 protein levels, and inhibition of the miRNA increases p-27 expression. p27 is a cyclin-dependent kinase inhibitor that blocks the cell cycle and accumulates the cells in the G0/G1 phase until mitogenic signals lead the cell to the S-phase (Chu et al. 2008). High p27 levels arrest the cell cycle in G1 -phase (Kullmann et al. 2013). Our findings are in concordance with the bibliography and miR-326 is suppressing the inhibitor of G1-S transition and allows the cells to reach the DNA synthesis phase (S-phase) without regulation. Furthermore, the bibliography has described that miR-326 overexpression increases the sub-G1 cell proportion and decreases the G0/G1, S, and G2/M cell proportions by reducing Cyclin D1 and Cyclin D2 levels in breast cancer (Ghaemi et al. 2019). In gastric cancer, overexpressed miR-326 blocks the G2/M phase due to the effect of the miRNA on the cell cycle regulator NOB1 (Ji et al. 2017). In colorectal cancer, miR-326 overexpression arrests the cell cycle in HCT116 and SW620 cells, increasing the G1-number of cells targeting NOB1 (Wu et al. 2015). Up to our knowledge, none reports have found relation between miR-326 expression and p-27 levels. For the first time, we have demonstrated that miR-326 overexpression leads to a decrease in the inhibition of the G0/G1 phase and increases the proportion of cells reaching the S-phase by targeting the kinase-inhibitor p-27 in Caco-2 cells.

Epithelial-mesenchymal transition is another highly repetitive process that carry out cancer cells and is characterised by the loss of cell adhesion ability and the acquisition of motility and invasive capacity. Well-established transcription factors such as ZEB1/2, SNAI1/2 and TWIST1/2 mediate the EMT process in response to signalling factors and these inducers can downregulate E-cadherin and upregulate N-cadherin and Vimentin, between others (Shibue et al. 2017). Our results have demonstrated that miR-326 overexpression reduces TWIST1, TWIST2 and SNAI1 genes, and inhibition of the miRNA promotes the opposite effect increasing the three transcription factors. At the same time, overexpression of the miRNA increased E-cadherin levels. These results suggest that miR-326 is regulating colorectal cancer progression process repressing some mesenchymal genes such as TWIST1, TWIST2 and SNAI1 and increasing epithelial genes like E-cadherin in Caco-2 cells. Related to that, some publications have described that miR-326 regulates EMT genes in lung carcinoma by targeting ADAM17 and ZEB1 genes (Cai et al. 2015; Liu et al. 2021). Furthermore, miR-326 regulates EMT in endometrial cancer by controlling TWIST1 (Liu et al., 2017). But up to our knowledge, no existing publication has characterized the role of miR-326 modulating EMT transcription factors in colorectal cancer cells. These findings are both in concordance and highlight miR-326 as a tumour suppressor miRNA by reducing proliferation process and reducing mesenchymal genes and increasing epithelial ones.

Furthermore, we have identified here for the first time ITGA5 as a direct target of miR-326 in Caco-2 cells. We validated the direct binding of miR-326 to ITGA5 3'-UTR by dual-luciferase assay. Moreover, ITGA5 exhibited an inverse correlation with miR-326 expression in our colorectal cancer patients. High levels of the miRNA correlated with low levels of ITGA5. This data suggest ITGA5 behaves as a direct target of miR-326 in tumour biopsies. As we have already reported, miR-326 expression decreases along the CRC disease and high levels of the miRNA are found in the first stages and low levels of miR-326 correlate with the late stages of the disease. In concordance with our findings, low levels of ITGA5 are found in the first stages of CRC and high levels in the late stages, when cancer is more aggressive and invasive. These results agree with the bibliography where ITGA5 is reported to be upregulated in many kinds of cancers. A recent publication reports that high ITGA5 expression is associated with lymph node metastasis in gastric cancer patients (Zhu et al. 2021). Furthermore, low ITGA5 expression leads the colorectal cancer cells to a less malignant phenotype, reducing cancer cell invasion and metastasis (Lu et al., 2019; Yu et al., 2019; Zhu et al., 2021). All these data together suggest miR-326 may act as an essential regulator of tumour progression in tumour primary biopsies of colorectal cancer patients and miR-326-ITGA5 axis could be pointed as a therapeutic target in the CRC context.

Furthermore, as ITGA5 associates with bad prognostic, we determined whether miR-326 was associated with fibronectin, the specific ligand of ITGA5. Fibronectin is one of the proteins constituting the extracellular matrix that participates in the earliest steps in primary tumour formation and the promotion of invasive tumour behaviour. It is widely accepted that FN levels are increased in several tumour types and this upregulation relates to the poor prognosis of the disease (Efthymiou et al. 2020; Wang et al. 2017). To that, cell adhesion to fibronectin assays were carried out in Caco-2 cells, considering fibronectin as a malignant tumoral matrix linked to tumour progression (Spada 2021). We described a decreased cell adhesion to the fibronectin matrix when miR-326 was overexpressed. Inhibition of miR-326 expression induced an increased tendency to fibronectin cell adhesion. Moreover, a recent publication has reported that fibronectin confers chemoresistance in breast cancer cells and overexpression of miR-326 reverts this effect by regulating ITGA5 as principal receptor of FN (Assidicky et al. 2022). These findings are in concordance with our results and support the importance of miR-326/ITGA5/FN in the progression of tumours. Overall, we conclude for the first time that miR-326 modulation affects the cell adhesion to malignant fibronectin most probably regulating ITGA5 expression in Caco-2 cells.

Furthermore, miR-326 regulates SNAIL expression in Caco-2 cells. Overexpression of the miRNA decreases SNAIL and inhibition of miR-326 levels increases SNAIL expression. SNAIL is a biomarker of poor prognosis in metastatic cancers and regulates integrin expression. High levels of SNAIL correlates with high levels of ITGA5 expression in prostate cancer cells (Neal et al. 2011). Moreover, SNAIL upregulates fibronectin, and this association is related to increased migration, invasion and metastasis (Kaufhold et al. 2014). We have previously described that miR-326 regulates SNAI1 expression gene and our results confirm that this miRNA regulates SNAIL protein levels too. Overall, we can confirm miR-326 is regulating cell adhesion to malignant fibronectin through the axis ITGA5/SNAIL, downregulating the expression levels of these proteins to avoid a progression of colorectal cancer in Caco-2 cells.

Apart from ITGA5, we have identified CXCR4 as another direct target of miR-326 in Caco-2 cells. The microRNA modulates the CXCR4 expression and miR-326 directly binds to CXCR4 3'UTR. Furthermore, we demonstrated that CXCR4 expression shows an inverse correlation with miR-326 levels in our colorectal cancer patients. These data suggest that CXCR4 represents a relevant prognostic indicator in tumour primary biopsies of colorectal cancer progression. As it was described in the introduction of this work, CXCR4 is involved in tumour growth and metastasis in various gastrointestinal cancers and is clearly associated with progression, TME, angiogenesis, poor survival, increased risk of death and metastasis in colorectal cancer (Chatterjee et al. 2014; Murakami et al. 2013; Ottaiano et al. 2021). In fact, CXCR4 expression is a prognostic factor for several gastrointestinal tumors and CXCR4 inhibitors are being studied in several clinal trials to treat colorectal cancer patients nowadays (Khare et al. 2021). Taken all these findings together, we can conclude that miR-326 and its target CXCR4 can be regulating the tumour colorectal cancer cells and the miR-326/CXCR4 axis could be pointed as a good therapeutic target for CRC patients' treatment.

On the other hand, miR-326 localizes in inflammatory cellularity, more specifically in NK cells and M1 macrophages. Based on that, we studied miR-326 expression in murine macrophage polarization (M1 and M2 macrophages) which behave differently in inflammation-related diseases. M1 macrophages are characterized by the production of pro-inflammatory cytokines, antimicrobial and tumoricidal activity. Conversely, M2 macrophages are part of immunosuppression, tumorigenesis, wound repair and elimination of parasites (Essandoh et al. 2016). Our *in vitro* experiments have demonstrated, in murine macrophages stimulated toward these two different phenotypes, that miR-326 is differently expressed in the cells depending on the stimulus received. M1 macrophages presented a low miR-326 expression and high expression of the miRNA was found in M2 macrophages.

Furthermore, the overexpression of miR-326 in an M2 context, induces an increase of the M1 proinflammatory macrophage marker (NOS2). miR-326 modulation did not affect NOS2 expression in Mφ and M1 contexts. On the other hand, the inhibition of the miRNA in an M2 context decreases the M2 macrophage marker (YM1 marker). miR-326 modulation did not affect YM1 expression in Mφ and M1 contexts. The discrepancy in the results suggest that miR-326 is not regulating macrophages polarization because the overexpression and the inhibition of the miRNA leads to the same effect, both pre-miR-326 and anti-miR-326 are trying to shift an M2 to an M1 phenotype in an M2 environment. This outcome suggests that the miRNA leads to a pro-inflammatory macrophage phenotype being the M1 a good prognostic marker of tumour progression. Taking this in consideration, exogenous modulation of the miRNA could be considered as a therapeutic target option for trying to revert an immune malignant tumour context. These results agree with the correlation found in our patients' biopsies where miR-326 correlated with M1 (CD68) macrophages and not with M2 (CD163) marker.

Based on the above, we conclude the following:
1. miR-326 presents a differential expression between healthy tissues and colorectal cancer tumours of patients. Moreover, higher levels of miR-326 correlate with different histopathologic features like left-side location, low stroma percentage, abundant immune infiltrate, low stage of tumour, low tumour invasion, absence of lymph nodes affectation and longer overall survival.
2. miR-326 is localized in different cell types within of the colorectal tumour biopsies such as epithelial tumour cells, fibroblasts, inflammatory cells and endothelium. Additionally, miR-326 positively correlates with macrophages and natural killer cells from immune system of colorectal cancer patients.
3. Overexpression of miR-326 reduces cell proliferation, cell adhesion to a tumourextracellular matrix and the expression of mesenchymal-transition genes, strongly suggesting the role of miR-326 in tumour progression.
4. M1 and M2 macrophage phenotypes present a different expression of miR-326, and this miRNA is a mediator of macrophage polarization and promotes NK pro-inflammatory phenotype.

Therefore, a first aspect of the invention refers to the use of miR-326 expression in tumour biopsies of CRC as a prognostic biomarker of CRC, preferably of the histopathological features of CRC tumors such as tumour location, stromal component, and presence of inflammatory infiltrate, all related differently to disease progression and prognosis. In particular, the use, preferably by qRT-PCR, of miR-326 expression in primary tumour biopsies is associated with overall survival (OS) as shown in the examples. As can be observed in Figure 6, expression levels of miR-326 were associated with the patient's overall survival. High levels of miR-326 were significantly associated with better patients' OS. This outcome agrees with the ones described in Figures 4 and 5 since high levels of miR-326 correlate with LCRC, early tumour stages, and pronounced immune infiltrate, parameters related to a good prognosis of the disease. Conversely, lower levels of miR-326 correlate with higher tumour stroma percentage and advanced tumour stages. All these findings suggest miR-326 could be used as a prognostic biomarker (Fig. 6).

A second aspect of the invention refers to the use of miR-326 expression in tumour biopsies of CRC for differentiating between tumoral and non-tumoral tissue, indicating that CRC associates with a decrease in miR-326 expression.

A third aspect of the invention refers to a composition comprising, in a preferably therapeutically effective amount, macrophages and/or natural killer (NK) cells, wherein these cells have been generated by transforming, transducing or transfecting these cells with miR-326, the seed sequence of miR-326 or a precursor of miR-326, or with a vector, linear piece of DNA or plasmid comprising a sequence coding for miR-326 or a precursor of miR-326, wherein the transformation, transduction or transfection is capable of significantly increasing the intracellular content or expression of miR-326 or the precursor of miR-326, transiently or permanently, in comparison to an untreated control macrophage or NK, and optionally simultaneously or subsequently to the transformation, transduction or transfection culturing these cells in the presence of a pro-inflammatory medium; for use in the treatment of cancer in a subject in need thereof.

It is noted that said transduction, transformation or transfection in NK cells provides for an increased cytotoxicity characterized by a significant increased expression of cytotoxicity activation marker NKG2C and preferably, a significantly decreased expression of the cytotoxicity inhibition markers CD158b and CD158e with respect to an untreated control NK which does not express miR-326, the seed sequence of miR-326 or a precursor of miR-326 or that only expresses basal levels of these sequences.

Therefore, a preferred embodiment refers to a composition comprising, in a preferably therapeutically effective amount, natural killer (NK) cells, wherein these cells have been generated by transforming, transducing or transfecting these cells with miR-326, the seed sequence of miR-326 or a precursor of miR-326, or with a vector comprising a sequence coding for miR-326, the seed sequence of miR-326 or a precursor of miR-326, wherein the transformation, transduction or transfection is capable of significantly increasing the intracellular content or expression of miR-326, the seed sequence of miR-326 or a precursor of miR-326, transiently or permanently, in comparison to an untreated control NK, wherein said transduction, transformation or transfection in NK cells provides for an increased cytotoxicity characterized by a significant increased expression of cytotoxicity activation marker NKG2C and preferably a significantly decreased expression of the cytotoxicity inhibition markers CD158b and CD158e with respect to an untreated control NK which does not express miR-326, the seed sequence of miR-326 or a precursor of miR-326 or that only expresses basal levels of these sequences.

It is further noted that said transduction, transformation or transfection in macrophages provides for an increased predisposition for these cells to shift from an M2 phenotype to a M1 phenotype when exposed or culture in a pro-inflammatory context or medium. In the context of the present invention, inflammatory context is understood as a tumour microenvironment (stromal cells that are comprised of fibroblasts and vascular cells and inflammatory immune cells), where cytokines can modulate an antitumoral response, or can also induce cell transformation and malignancy, conditional on the balance of a pro-tumoral response. The most common cytokines involved in an inflammatory context are TNF-a, IL-6, TGF-β and IL-10. For mimicking this inflammatory context some or all of these cytokines are supplemented to the culture media. Therefore, culturing in the presence of an inflammatory context is understood as culturing the cells in a suitable culture medium that mimics the inflammatory context, wherein said culture medium comprises cytokines involved in an inflammatory context selected from the list consisting of TNF-a, IL-6, TGF-β and/or IL-10, or any combination thereof. It is noted that for macrophage differentiation some non-limiting examples of suitable culture medium would be an RPMI medium containing or comprising 10% of FBS, 100 U/ml Penicillin-Streptomycin, 2,5% of HEPES Solution Buffer pH 7.5, and 10 ng/ml of Recombinant Mouse M-CSF. For macrophage Polarization from M2 to M1 would be completed RPMI media in which polarization factors were added. Cells can be for example stimulated with LPS (10 ng/ml) and IFN-γ (10 ng/ml) to promote M1 phenotype and IL-4 (10 ng/ml) to promote M2 phenotype.

It is noted that in a preferred embodiment, the vector, linear piece of DNA or plasmid comprising a sequence coding for miR-326, the seed sequence of miR-326 or a precursor of miR-326 and capable of significantly increasing the intracellular expression of miR-326, the seed sequence of miR-326 or a precursor of miR-326, comprises an expression construct to be carried into the cell by said vector, linear piece of DNA or a plasmid. In particular, the construct coding for miR-326, the seed sequence of miR-326 or a precursor of miR-326, is characterized by comprising at least one or more transcription promoter sequences, one or more ORFs encoding the miR-326, the seed sequence of miR-326 or a precursor of miR-326, and one or more transcription termination sequences. The promoter sequence might be any known sequence in the state of the art able to promote transcription in an epithelial cell and/or immune cell such as a macrophage or a NK cell. The expression construct might comprise several ORFs in tandem, each one flanked by a promoter and a termination, transcriptional termination, sequence upstream and downstream of the ORF, respectively, therefore built for allowing independent expression of each ORF, controlled by a specific promoter. Alternatively, the expression construct might have one or several ORFs within an operonlike structure with polycistronic expression controlled by a common promoter.

In a preferred embodiment, the miR-326 is hsa-miR-326 MIMAT0000756 of SEQ ID NO 1: **CCUCUGGGCCCUUCCUCCAG, or**
of any nucleotide sequence that has at least 80%, 85%, 90%, 91%, 92%, 93%, 94%, 95%, 96%, 97%, 98%, or 99% identity with the full/length sequence of SEQ ID No. 1.

In another embodiment, the miR-326 is at least 10, 11, 12, 13, 14, 15, 16, 17, 18, 19, 20 nucleotides in length. Preferably, the miR-326 comprises, consists, or consists essentially of a fragment composed of a succession of at least 7, 8, 9, 10, 11, 12, 13, 14, 15, or 16 consecutive nitrogen bases of nucleotide or nucleotide analogue units that are identical in at least 60%, 70%, 80%, 85%, 90%, 91%, 92%, 93%, 94%, 95%, 96%, 97%, 98%, 99% or 99.5%, or 100% to SEQ ID NO 1. More preferably, the sequence of the nitrogen bases of the nucleotide or nucleotide analogue units comprised in the miR-326 is identical in at least 60%, 70%, 80%, 85%, 90%, 91%, 92%, 93%, 94%, 95%, 96%, 97%, 98%, 99% or 99.5%, or 100% to SEQ ID NO 1.

In another preferred embodiment, the miR-326 is the seed of hsa-miR-326 MIMAT0000756 of SEQ **ID NO 3: CUCUGGG,** or
of any nucleotide sequence that has at least 70%, 80%, 85%, 90%, 91%, 92%, 93%, 94%, 95%, 96%, 97%, 98%, or 99% identity with the full/length sequence of SEQ ID No. 3.

In another preferred embodiment, the precursor of miR-326 is hsa-miR-326 MI0000808 of **SEQ ID NO 2:** 
CUCAUCUGUCUGUUGGGCUGGAGGCAGGGCCUUUGUGAAGGCGGGUGGUGCUCAGAUCGCCUCUGG
GCCCUUCCUCCAGCCCCGAGGCGGAUUCA,
 or of any sequence that has at least 80%, 85%, 90%, 91%, 92%, 93%, 94%, 95%, 96%, 97%, 98%, or 99% identity with the full/length sequence of SEQ ID No. 2.

In another embodiment, the precursor of miR-326 is at least 10, 11, 12, 13, 14, 15, 16, 17, 18, 19, 20, 25, 30, 35, 40, 45, 50, 55, or 60 nucleotides in length. Preferably, the precursor miR-326 comprises, consists, or consists essentially of a fragment composed of a succession of at least 7, 8, 9, 10, 11, 12, 13, 14, 15, 20, 25, 30, 35, 40, 45, 50, 55, or 60 consecutive nitrogen bases of nucleotide or nucleotide analogue units that are identical in at least 60%, 70%, 80%, 85%, 90%, 91%, 92%, 93%, 94%, 95%, 96%, 97%, 98%, 99% or 99.5%, or 100% to SEQ ID NO 2. More preferably, the sequence of the nitrogen bases of the nucleotide or nucleotide analogue units comprised in the precursor of miR-326 is identical in at least 60%, 70%, 80%, 85%, 90%, 91%, 92%, 93%, 94%, 95%, 96%, 97%, 98%, 99% or 99.5%, or 100% to SEQ ID NO 2.

The terms "sequence identity" or "percent identity" in the context of the nucleotide sequences indicated through-out the present invention, refers to two or more sequences or subsequences that are the same ("identical") or have a specified percentage of nucleotides that are identical ("percent identity") when compared and aligned for maximum correspondence with a second molecule, as measured using a sequence comparison algorithm (e.g., by a BLAST alignment, or any other algorithm known to persons of skill), or alternatively, by visual inspection.

In the context of the present invention, M1 phenotype is also called classically activated macrophages or pro-inflammatory macrophages. They are differentiated from monocytes mainly by granulocytemacrophage colony-stimulating factor (GMCSF) and activated by interferon-y (IFNy) mostly released by lymphocytes, lipopolysaccharide (LPS) through toll-like receptor-4 (TLR4), Th1 cytokines, such as tumour necrosis factor alpha (TNF-α), and colony stimulating factor 2 (CSF2). M1 macrophages are characterized by their high expression of proinflammatory molecules includingTNFα, HLA-DR, CD11c, CD86, pSTAT1, IL-1β, IL-6, IL-12, IL-13, and ROS (nitric oxygen, NO, and inducible nitric oxide synthase, NOS2).

In the context of the present invention, M2 phenotype is also known as alternatively activated macrophages or anti-inflammatory macrophages. They are differentiated from Mφ or M1 macrophages byTh2-derived cytokines such as IL4, IL10, IL13, transforming growth factor beta (TGFβ) or prostaglandin E2 (PGE2). M2 phenotype have signatures including high IL-10 and low IL-12 cytokine expression levels, suppressor of cytokine signaling-3 (SOC3), macrophage colony-stimulating factor (M-CSF), CD206, found in inflammatory zone-1 (Fizz1 or RENTLA), chitinase-3-like protein 3 (Ym1 or CD163) and arginase-1 (Arg1), CD204, VEGF and cMAF.

In the context of the present invention, NK or natural killer NK cells are innate lymphocytes that belong to the rapidly expanding family of innate lymphoid cells (ILC) and represent 5-20% of all circulating lymphocytes in humans. NK cell is a specialized immune effector cell type that plays a critical role in immunity against viruses and in the immune surveillance of tumors. NK cells can be identified by the presence of CD56 and the absence of CD3 (CD56+, CD3-). NK cells exert their cytotoxic activity through the secretion of granzyme and perforin. Another mechanism of regulation of this cytotoxic activity is through activation receptors such as NKG2D and NKG2C or inhibitory receptors such as NKG2A, CD158a, CD158b and CD158e. CD16 (FcylllA) plays a role in antibodydependent cell-mediated cytotoxicity.

Macrophage. Macrophages are a subtype of leukocytes and constitute crucial cells of innate immunity implicated in homeostasis maintenance and inflammation response. They are recruited to the inflammation site, as blood peripheral monocytes that scape from the blood flow and migrate into tissues, where they differentiate into macrophages responding to specific signalling. The most relevant characteristic of macrophages is their diversification capacity and functional plasticity in response to specific stimuli from the microenvironment, which has been described as macrophage polarization in different phenotypes. Conventionally, the major phenotypes reported are naive macrophages (Mφ), M1 pro-inflammatory macrophages, and M2 anti-inflammatory macrophages.

miR-326. It is a non-coding RNA involved in post-transcriptional regulation of gene expression in multicellular organisms by affecting both the stability and translation of mRNAs. miR-326 is transcribed by RNA polymerase II as part of capped and polyadenylated primary transcript (pri-miR-326) that is cleaved by the Drosha ribonuclease III enzyme to produce an approximately 70-nt stemloop precursor miRNA (pre-miRNA), which is further cleaved by the cytoplasmic Dicer ribonuclease to generate the mature miRNA and antisense miRNA product. The mature miR-326 is incorporated into an RNA-induced silencing complex (RISC), which recognizes target mRNAs through imperfect base pairing with the miRNA and most commonly results in translational inhibition or destabilization of the target mRNA. miR-326 is defined by nucleotide sequence as indicated above.

Precursor of miR-326. The precursor of miR-326 is the resulting RNA derived from the primary transcript termed pri-miR-326. The pre-miR-326 is delivered to the cytoplasm via a process that involves Exportin-5. In cytoplasm, the pre-miR-326 is further cleaved by the RNase III enzyme Dicer to generate the double-stranded RNA miR-326. The precursor of miR-326 is defined by nucleotide sequence as indicated above.

Significantly increasing the intracellular expression or content of miR-326, the seed sequence of miR-326 or a precursor of miR-326 in comparison to an untreated control macrophage or NK, is understood as the relative expression or content of miR-326, the seed sequence of miR-326 or a precursor of miR-326 between a control of non-expression such as pre-miR-Scrambled and the expression or content of miR-326, the seed sequence of miR-326 or a precursor of miR-326 in a transformed, transfected or transduced NK or macrophage, where the p-value is preferably lower than 0.05, preferably lower than 0.01, much preferably lower than 0.001. The increase of this expression or content may also be understood as of more than 2, 3, 4, 5, 6, 7, 8, 9 or preferably 10-fold times the expression or content of miR-326, the seed sequence of miR-326 or a precursor of miR-326 relative to the expression or content of this marker in a non-expressing cell such as an untreated control macrophage or NK which do not express miR-326, the seed sequence of miR-326 or a precursor of miR-326 or express basal levels of these sequences. Expression can be for example measured by qPCR.

Significantly increasing the expression of the cytotoxicity activation marker NKG2C is understood as the relative expression of NKG2C between an NK cell having a significant expression or content of miR-326, the seed sequence of miR-326 or a precursor of miR-326, in comparison to an untreated NK cell not expressing miR-326, the seed sequence of miR-326 or a precursor of miR-326 or expressing basal values of these sequences, wherein preferably the p-value is lower than 0.05, preferably lower than 0.01, much preferably lower than 0.001. The increase of this expression may also be understood as of more than 2, 3, 4, 5, 6, 7, 8, 9 or preferably 10-fold times the expression of NKG2C relative to the expression of this marker in a non-expressing cell such as an untreated control NK which does not express miR-326, the seed sequence of miR-326 or a precursorof miR-326 or basal levels of these sequences.

In a preferred embodiment of the third aspect of the invention or of any of its preferred embodiments, the cells are transformed, for example as indicated in the examples of the present specification, with miR-326, the seed sequence of miR-326 or a precursor of miR-326 as defined above. In the context of the present invention, the transformation is preferably carried out by transfection by lipofectamine. In this sense, just as a non-limiting example, the transfection is preferably performed when cells reach 70-80% confluence. First, lipofectamine is incubated with Opti-MEM for 10 minutes. Additionally, pre-miRs are diluted in Opti-MEM at appropriated concentration. After, preferably 10 minutes, at room temperature, the lipofectamine is added to miRNAs and incubated to allow the formation of transfection complexes. Cell cultures are washed twice with 1X PBS, and one last time with Opti-MEM. After 20 minutes of incubation, the mixes of each condition were added to the cell culture. 5 hours later, DMEM was added and incubated overnight. After 18h, the culture medium was removed from the plates, and complete DMEM medium was added. Cells were kept in culture for up to 24-, 48- or 72-hours post-transfection depending on the experiment.

Also preferably, the vector use to transduced or transfect the cells is a viral vector such as lentivirus, adenoviruses or adenoassociated viruses, polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates), dry powder formulations, plasmids, viruses, calcium phosphate nucleotides, aptamers, peptides and other vectorial tags.

In another preferred embodiment of the third aspect of the invention or of any of its preferred embodiments, the composition comprising macrophages is simultaneously or subsequently to the transformation, transduction or transfection of the cells, culture in the presence of a proinflammatory medium to increase the proportion of the M1 phenotype in the macrophages present in the composition, said proportion is increased in at least 1%, 5%, 10% or at least 20%, that is the number of M1 macrophages is increased in at least 1%, 5%, 10% or at least 20%, in comparison to the number of M1 macrophages present prior to the culturing step in the presence of a proinflammatory medium.

In another preferred embodiment of the third aspect of the invention or of any of its preferred embodiments, said macrophages or natural killer (NK) cells are human. Preferably, said macrophages or natural killer (NK) cells are autologous or allogeneic to a human subject having cancer and treated with the composition of the present invention.

In another preferred embodiment of the third aspect of the invention or of any of its preferred embodiments, the disease or disorder is colorectal cancer.

A fourth aspect of the invention refers to a composition comprising the therapeutically effective amount of macrophages and/or natural killer (NK) cells as defined in the third aspect of the invention. Preferably, the composition is a pharmaceutical composition optionally further comprising a carrier and/or adjuvant.

The present invention thus provides, among other things, methods and compositions for the effective therapeutical treatment of cancer, preferably of CRC.

A fifth aspect of the invention refers to the composition of the fourth aspect for use in therapy.

A sixth aspect of the invention refers to a therapeutically effective amount of a composition comprising a miR-326, the seed sequence of miR-326 or a precursor of miR-326, or a delivery vector of miR-326, the seed sequence of miR-326 or a precursor of miR-326 such as a plasmid or vector comprising a sequence coding for miR-326, the seed sequence of miR-326 or a precursor of miR-326, wherein said composition is capable of significantly increasing the intracellular concentration of miR-326, the seed sequence of miR-326 or a precursor of miR-326 in the macrophages or natural killer (NK) cells of a subject in need thereof in comparison to an untreated control macrophage or NK; for use in the treatment of cancer.

It is noted that also in the context of the sixth aspect of the invention (and in the context of the present full specification), miR-326, the seed sequence of miR-326 or a precursor of miR-326 are as defined in the third aspect of the invention.

In a preferred embodiment of the sixth aspect of the invention or of any of its preferred embodiments, said composition comprises a delivery vehicle of miR-326, the seed sequence of miR-326 or a precursor of miR-326. Preferably, the delivery vehicle is selected from the list consisting of viral vectors such as lentivirus, adenoviruses or adenoassociated viruses, polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates), dry powder formulations, plasmids, viruses, calcium phosphate nucleotides, aptamers, peptides and other vectorial tags. More preferably, the delivery vehicle is a viral vector selected from the list consisting of lentivirus, adenoviruses or adenoassociated viruses. More preferably, the delivery vehicle is a liposome or lipid carrier or nanocarrier.

In another preferred embodiment of the sixth aspect of the invention or of any of its preferred embodiments, said composition is administered by a route selected from the list consisting of oral, rectal, vaginal, transmucosal, or intestinal administration, parenteral delivery, including intradermal, transdermal (topical), intramuscular, subcutaneous, intramedullary injections, as well as intrathecal, direct intraventricular, intravenous, intraperitoneal, or intranasal.

In another preferred embodiment of the sixth aspect of the invention or of any of its preferred embodiments, the disease or disorder is colorectal cancer and the treatment at the effective therapeutic dose is carried out at an administration interval such that at least one symptom or feature of colorectal cancer is reduced in intensity, severity, or frequency or has delayed in onset.

Finally, the invention further refers to a method of production of a composition comprising macrophages and/or natural killer (NK) cells, wherein the method comprises the steps of transforming, transducing or transfecting these cells in vitro with miR-326, the seed sequence of miR-326 or a precursor of miR-326, or with a vector comprising a sequence coding for miR-326, the seed sequence of miR-326 or a precursor of miR-326, wherein the transformation, transduction or transfection is capable of significantly increasing the intracellular expression of miR-326, the seed sequence of miR-326 or a precursor of miR-326, transiently or permanently, in comparison to an untreated control macrophage or NK, and optionally, with the proviso that if the composition comprises macrophages, simultaneously or subsequently to the transformation, transduction or transfection culturing these cells in the presence of a pro-inflammatory medium to increased the proportion of M1 macrophages according to the invention. Preferably, the composition comprises natural killer (NK) cells, and the method comprises the steps of transforming, transducing or transfecting these cells with miR-326, the seed sequence of miR-326 or a precursor of miR-326, or with a vector comprising a sequence coding for miR-326, the seed sequence of miR-326 or a precursor of miR-326, wherein the transformation, transduction or transfection is capable of significantly increasing the intracellular content or expression of miR-326, the seed sequence of miR-326 or a precursor of miR-326, transiently or permanently, in comparison to an untreated control NK, wherein said transduction, transformation or transfection in NK cells provides for an increased cytotoxicity characterized by a significant increased expression of cytotoxicity activation marker NKG2C with respect to an untreated control NK which does not express miR-326, the seed sequence of miR-326 or a precursor of miR-326 or that expresses basal levels of these sequences.

Preferably, for the method of the present invention, the vector is a viral vector such as lentivirus, adenoviruses or adenoassociated viruses, polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates), dry powder formulations, plasmids, viruses, calcium phosphate nucleotides, aptamers, peptides and other vectorial tags.

Preferably, said macrophages or natural killer (NK) cells are of human origin. More preferably, said macrophages or natural killer (NK) cells are autologous or allogeneic to a human subject which is going to be subjected to a cancer treatment.

To facilitate expression of miR-326 agents, such as miR-326, the seed sequence of miR-326 or a precursor of miR-326, in vivo, any of the delivery vehicles above mentioned, such as liposomes or viral carriers, can be formulated in combination with one or more additional nucleic acids, carriers, targeting ligands or stabilizing reagents, or in pharmacological compositions where it is mixed with suitable excipients. Techniques for formulation and administration of drugs may be found in "Remington's Pharmaceutical Sciences," Mack Publishing Co., Easton, Pa., latest edition.

Provided liposomally-encapsulated or associated miR-326 agents and compositions containing the same, may be administered and dosed in accordance with current medical practice, taking into account the clinical condition of the subject, the site and method of administration, the scheduling of administration, the subject's age, sex, body weight and other factors relevant to clinicians of ordinary skill in the art. The "effective amount" for the purposes herein may be determined by such relevant considerations as are known to those of ordinary skill in experimental clinical research, pharmacological, clinical and medical arts. In some embodiments, the amount administered is effective to achieve at least some stabilization, improvement or elimination of symptoms and other indicators as are selected as appropriate measures of disease progress, regression or improvement by those of skill in the art.

As used herein, the term "therapeutically effective amount" is largely determined based on the total amount of the therapeutic agent contained in the pharmaceutical compositions of the present invention. Generally, a therapeutically effective amount is sufficient to achieve a meaningful benefit to the subject (e.g., treating, modulating, curing, preventing and/or ameliorating cancer). For example, a therapeutically effective amount may be an amount sufficient to achieve a desired therapeutic and/or prophylactic effect. Generally, the amount of the miR-326 agents, such as miR-326 or precursors of miR-326, administered to a subject in need thereof will depend upon the characteristics of the subject. Such characteristics include the condition, disease severity, general health, age, sex and body weight of the subject. One of ordinary skill in the art will be readily able to determine appropriate dosages depending on these and other related factors. In addition, both objective and subjective assays may optionally be employed to identify optimal dosage ranges.

A therapeutically effective amount is commonly administered in a dosing regimen that may comprise multiple unit doses. For any particular therapeutic protein, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the patient; the time of administration, route of administration, and/or rate of excretion or metabolism of the specific protein employed; the duration of the treatment; and like factors as is well known in the medical arts.

The terms "modulate," "modulation," "modify," "modulator," "modifier" and the like refer to the ability of a compound to increase the activity and/or expression of miR-326, where such function may include transcription regulatory activity and/or nucleic acid-binding.

The "subject" is defined herein to include animals such as mammals, including, but not limited to, primates (e.g., humans), cows, sheep, goats, horses, dogs, cats, rabbits, rats, mice and the like. In some prefer embodiments, the subject is a human.

The terms "treat," "treating," "treatment" and grammatical variations thereof as used herein, include partially or completely delaying, alleviating, mitigating or reducing the intensity of one or more attendant symptoms of a disorder or condition and/or alleviating, mitigating or impeding one or more causes of a disorder or condition. Treatments according to the invention are applied preventively, prophylactically, palliatively or remedially.

The term "administering" refers to oral administration, administration as a suppository, topical contact, intravenous, intraperitoneal, intramuscular, intralesional, intranasal or subcutaneous administration, or the implantation of a slow-release device e.g., a mini-osmotic pump, to a subject. Administration is by any route, including parenteral and transmucosal (e.g., buccal, sublingual, palatal, gingival, nasal, vaginal, rectal, or transdermal). Parenteral administration includes, e.g., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, etc.

It is important to note that according to the present invention, miR-326, a pre- miR-326 or a miR-326 modulator as described herein may be delivered to the tumour of a subject in need thereof as naked RNA (unpackaged), in the case of miR-326 or pre-miR-326, or via delivery vehicles. As used herein, the terms "delivery vehicle," "transfer vehicle," "Nanoparticle" or grammatical equivalent, are used interchangeably.

In the present invention, we refer to the combination of terms miR-326, the seed sequence of miR-326 or a precursor of miR-326 as "miR-326 agents".

miR-326 agents according to the present invention may be synthesized according to any of a variety of known methods. For example, miRNAs according to the present invention may be synthesized via in vitro transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7 or SP6 RNA polymerase), DNAse I, pyrophosphatase, and/or RNAse inhibitor. The exact conditions will vary according to the specific application. In some embodiments, for the preparation of miRNA according to the invention, a DNA template is transcribed in vitro. A suitable DNA template typically has a promoter, for example a T3, T7 or SP6 promoter, for transcription, followed by desired nucleotide sequence for desired mRNA and a termination signal.

In some embodiments, miR-326 agents according to the present invention may be synthesized as unmodified or modified miRNA. Typically, miRNAs are modified to enhance stability. Modifications of miR-326 can include, for example, modifications of the nucleotides of the RNA. A modified miR-326 according to the invention can thus include, for example, backbone modifications, sugar modifications or base modifications. In some embodiments, miR-326 or a pre- miR-326 may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as e.g. 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, β-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art e.g. from the U.S. Pat. No. 4,373,071, U.S. Pat. No. 4,401,796, U.S. Pat. No. 4,415,732, U.S. Pat. No. 4,458,066, U.S. Pat. No. 4,500,707, U.S. Pat. No. 4,668,777, U.S. Pat. No. 4,973,679, U.S. Pat. No. 5,047,524, U.S. Pat. No. 5,132,418, U.S. Pat. No. 5,153,319, U.S. Pat. Nos. 5,262,530 and 5,700,642, the disclosures of which are incorporated by reference in their entirety.

In some embodiments, miR-326 or a pre- miR-326 may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (e.g. cytidine 5'-O-(1-thiophosphate)), boranophosphates, positively charged guanidinium groups etc., which means by replacing the phosphodiester linkage by other anionic, cationic or neutral groups.

In some embodiments, miR-326 or a pre- miR-326 may contain sugar modifications. A typical sugar modification is a chemical modification of the sugar of the nucleotides it contains including, but not limited to, sugar modifications chosen from the group consisting of 2'-deoxy-2'-fluorooligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide(2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In some embodiments, miR-326 or a pre- miR-326 may contain modifications of the bases of the nucleotides (base modifications). A modified nucleotide which contains a base modification is also called a base-modified nucleotide. Examples of such base-modified nucleotides include, but are not limited to, 2-amino-6-chloropurine riboside 5'-triphosphate, 2-aminoadenosine 5'-triphosphate, 2-thiocytidine 5'-triphosphate, 2-thiouridine 5'-triphosphate, 4-thiouridine 5'-triphosphate, 5-aminoallylcytidine 5'-triphosphate, 5-aminoallyluridine 5'-triphosphate, 5-bromocytidine 5'-triphosphate, 5-bromouridine 5'-triphosphate, 5-iodocytidine 5'-triphosphate, 5-iodouridine 5'-triphosphate, 5-methylcytidine 5'-triphosphate, 5-methyluridine 5'-triphosphate, 6-azacytidine 5'-triphosphate, 6-azauridine 5'-triphosphate, 6-chloropurine riboside 5'-triphosphate, 7-deazaadenosine 5'-triphosphate, 7-deazaguanosine 5'-triphosphate, 8-azaadenosine 5'-triphosphate, 8-azidoadenosine 5'-triphosphate, benzimidazole riboside 5'-triphosphate, N1-methyladenosine 5'-triphosphate, N1-methylguanosine 5'-triphosphate, N6-methyladenosine 5'-triphosphate, O6-methylguanosine 5'-triphosphate, pseudouridine 5'-triphosphate, puromycin 5'-triphosphate or xanthosine 5'-triphosphate.

The following examples merely illustrate the present invention but do not limit the same.

### Examples

### Materials and methods

Methods and materials used to address the objectives proposed in this invention will be described in this section. Conditions of each method are detailed below, and material used for each technique has been specified.

### COLORECTAL BIOPSIES COLLECTION: ETHICS STATEMENT

A total of 192 CRC paraffin-embedded tissue samples were collected from patients who underwent CRC surgery at the Ramon y Cajal University Hospital, Elche University Hospital and Virgen de la Victoria University Hospital. Biopsies were evaluated by a pathologist and staged according to the guidelines of the Union for International Cancer Control tumour-node-metastasis TNM staging system (AJCC/UICCTNM, 7th edition).

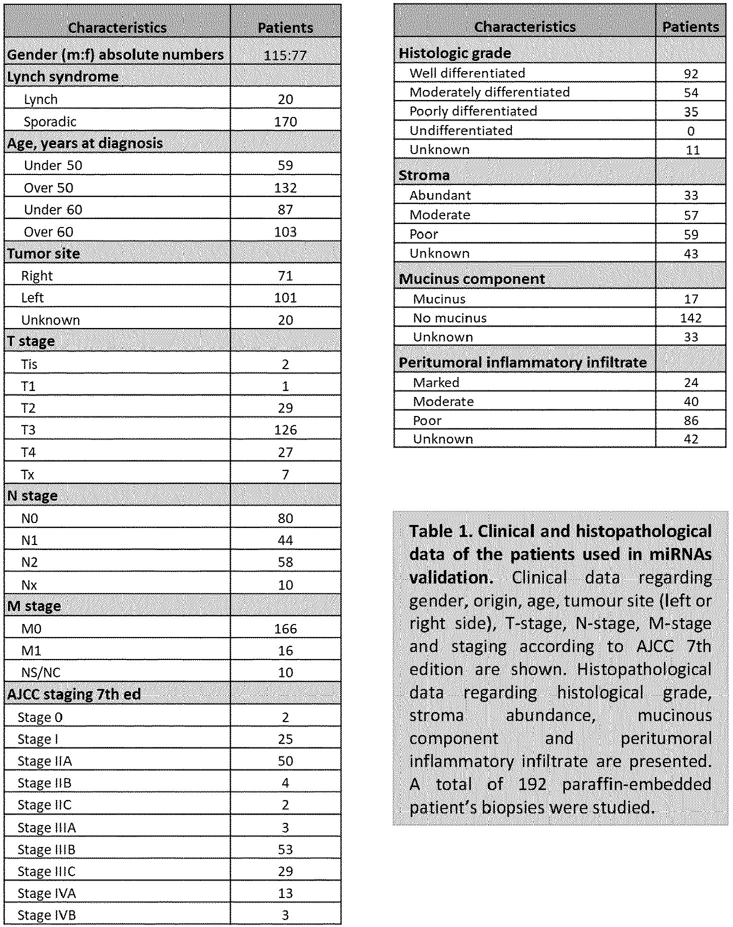

The ethics committee of the Ramón y Cajal University Hospital approved this study. The clinical and histopathological characteristics of this cohort are shown in Table 1. Tissue was obtained for molecular analysis via manually punches from paraffin-embedded tumour regions selected by the pathologist.

### COLORECTAL CANCER CELL LINES: CELL CULTURE

Different colorectal cancer cell lines have been used in this work:
- Caco-2: Epithelial colorectal adenocarcinoma cells derived from an intestine of 72 ages male. Caco-2 cells carry mutation in the TP53 gene.
- HT-29: Epithelial line isolated from a primary tumour derived from a 44-years female. HT-29 cells carry mutation in BRAF, PIK3CA and TP53 genes.
- SW620: Cell line derived from a lymph node metastasis of a primary tumour from a 51 aged male. SW620 cells carry mutation in KRAS and TP53 genes.
- RKO: Poorly differentiated colon carcinoma cell line. RKO cells carry mutation in BRAF and PIK3CA genes (Ahmed et al., 2013).
- CCD841: Epithelial colon cells isolated from normal human colon tissue. The cells resemble epithelial cells; however, the cells do not express keratin.
- MC-38: Tumorigenic epithelial cell line isolated from mice with colon adenocarcinoma. MC-38 cells are derived from a C57BL6 female mouse adenocarcinoma III, following prolonged exposure to the carcinogen DMH (1,2-dihydrochloride dimethylhydrazine)._The MC-38 cells were used to develop an *in vitro* MC-38 syngeneic model to overexpress miR-326 with lentiviral vector-based technologies for further *in vivo* studies.

Caco-2, HT-29, SW620, RKO and CCD841 from ATCC, were cultured in DMEM medium (#31885049, Life Technologies), supplemented with 10% FBS (#10099-141, Life Technologies) and 1% penicillin-streptomycin-glutamine (#10378016, Life Technologies). MC-38, from Kerafast, were cultured in DMEM medium supplemented with 10% FBS, 1% penicillin-streptomycin-glutamine, 0.1 mM nonessential amino acids (#11140-035, Life Technologies), 10 mM Hepes (#15630080, Life Technologies). Cultures were maintained in a regular incubator in a humidified atmosphere with 5% CO₂ at 37°C. Cells lines are adherent, and 0.25% Trypsin-1X EDTA (#25200072, Life Technologies) was used to detach adherent cells for further uses.

### Isolation and establishment of Murine Macrophages

To obtain primary murine macrophages differentiated from monocytes, monocytes were extracted from the bone marrow of the mouse femur and tibiae. For that, an incision in the midline of the abdomen was carried out in C57BL/6J males between 2-3 weeks of age. Skin and muscles were dissected from abdomen and hind legs. The 2 femurs and 2 tibiae were removed from the hind legs by cutting through the pelvis close to the hip joint with the aid of a scalpel and dissecting scissors.

The end of the bones was cut, and bones were collected in a 200 uL empty Eppendorf which was located in a 1,5 mL Eppendorf with 200 uL of completed RPMI medium. Tubes were centrifugated 30 seconds at room temperature at 7500 rpm in a microcentrifuge to pellet cells. Once the bone marrow was expelled the bone become transparent. After, cells were resuspended in fresh media to reach macrophages differentiation.

### Macrophages Differentiation

Macrophage differentiation media is RPMI (#10379144, Life Technologies) containing 10% of FBS (#DE14-801F, batch 9SB024, Lonza), 100 U/ml Penicillin-Streptomycin (#15070-063, Invitrogen), 2,5% of HEPES Solution Buffer pH 7.5 (#17-737E, Biowhittaker), and 10 ng/ml of Recombinant Mouse M-CSF (#315-02-B, PeproTech). After 5 days, the monocytes differentiate to macrophages. Erythrocytes and other unattached cells were removed by washing with PBS, and then media was replaced with fresh macrophage differentiation media.

### Macrophages Polarization

To induce polarization in the murine macrophages, approximately 4x10⁵ cells/well were seeded in 12 well plates and incubated for 24 hours at standard conditions with complete RPMI media in which polarization factors were added. Cells were stimulated with LPS (10 ng/ml) (#L2654, Sigma-Aldrich) and Murine IFN-γ (10 ng/ml) (#315-05, PeproTech) to promote M1 phenotype and IL-4 (10 ng/ml) (#214-14, Peprotech) to promote M2 phenotype.

### Modulation of miR-326 in a Human Colon Cancer Cell line: Caco-2

To overexpress and reduce miR-326 expression, this miRNA was modulated in Caco-2 cells by transient transfection with Pre-miR-326 (hsa-miR-326, miRVana^{™}, #4464066, microRNA mimic, Ambion) and Pre-miR-Scrambled (miRVana^{™}, microRNA mimic Negative Control, #4464088, Ambion) at a final concentration of 0.25 nM. Anti-miR-326 (hsa-miR-326, miRVana^{™}, microRNA Inhibitor, #4464084 Ambion) and Anti-miR-Scrambled (miRVana^{™}, microRNA Inhibitor Negative Control #4464077, Ambion) were used at a final concentration of 50 nM.

The transfection reagent and culture medium were Lipofectamine 2000 (#11668027, Invitrogen), and Opti-MEM (#31985054, Life Technologies), respectively. The transfection was performed in p60/p6-well plates when cells reach 70-80% confluence. First, lipofectamine was incubated with Opti-MEM for 10 minutes following the instructions of the transfection agent manufacturer. Additionally, pre-miRs and anti-miRs were diluted in Opti-MEM at the concentrations listed above. After 10 minutes at room temperature, the transfection reagent was added to miRNAs and incubated for 20 minutes to allow the formation of transfection complexes. The final transfection volume was adjusted to the size of the plate, p60-well transfection was performed in 2 ml and p6-well in 1 ml final volume. Cell cultures were washed twice with 1X PBS (#10010056, Life Technologies), and one last time with Opti-MEM. After 20 minutes of incubation, the mixes of each condition were added to the cell culture. 5 hours later, 2 ml or 1 ml (depending on well size) of full DMEM was added and incubated overnight. After 18h, the culture medium was removed from the plates to avoid excessive toxicity associated to lipofectamine, and complete DMEM medium was added. Cells were kept in culture for up to 24-, 48- or 72-hours post-transfection depending on the experiment, and supernatants and cells were harvested and stored at -80°C for further analyses. miR-326 expression was determined by qRT-PCR in order to confirm miR-326 modulation.

### Modulation of miR-326 in Murine Macrophages

miR-326 expression was modulated in cultured murine macrophages by transient transfection with Pre-miR-326 to overexpress miRNA at a final concentration of 10 nM, and Anti-miR-326 to inhibit the miRNA expression at a final concentration of 50 nM. Pre-miR-Scrambled and Anti-miR-Scrambled were used as negative control tools of transfection. It is important to mention that in this work, *in vitro* modulation of miR-326 expression was performed after murine macrophages were stimulated towards different phenotypes in p12-well plates. The transfection reagent and medium used were 2,5 µL/well of Lipofectamine 2000 and 500 µL/well of standard RPMI, respectively. Transfection procedure in macrophages followed the same protocol detailed above for epithelial cancer cells and miR-326 expression was determined by qRT-PCR in order to confirm miR-326 modulation.

### Modulation of miR-326 in a Murine Colon Cancer Cell line: MC-38

### Modulation by microRNA Mimics and Inhibitors

To overexpress and reduce miR-326 expression, this miRNA was modulated in MC-38 cells by transient transfection with Pre-miR-326 and Pre-miR-Scrambled at a final concentration of 0.25 nM. Anti-miR-326 and Anti-miR-Scrambled were used at a final concentration of 50 nM, following the same protocol described in the previous section carried out in Caco-2 cells.

### Modulation by Lentiviral Vectors

Lentiviral vector (LV)-based technologies allow the long-term overexpression of genes/microRNAs. To overexpress miR-326 expression for further *in vivo* experiments, Mouse pre-microRNA Expression Construct miR-326 (MMIR-326-PA-1) and Mouse precursor Scrambled negative control construct (MMIR-000-PA-1) were generated, amplificated and tittered by System Biosciences *(**Figure* 1). A second tittering was made in the laboratory to ensure the number of infectious virus particles of each condition. MMIR-326 was tittered as 9 × 10⁷ IFU/ml and MMIR-000 as 8,43 × 10⁷ compared to 2,09 × 10⁹ and 7,76 × 10⁹ from Biosciences' titters.

MOI (multiplicity of infection) is the number of viral particles that can infect each cell in the culture vessel. To calculate MC-38 MOl, 2×10⁵ cells were seeded in p-6 well plates with complete DMEM. The lentivirus stocks (MMIR-326-PA-1 and MMIR-000-PA-1) were diluted in serial dilutions from 1, 5, 10, 20, 50 and 100x into a final volume of 2 ml of complete DMEM containing 8 µg/ml of polybrene (#H9268, Sigma-Aldrich) to determine the MC-38 MOI. To calculate the volume of virus stock to add: [number of cells (2×10⁵) × MOI desired]/Virus titter (IFU/ml). A non-infected well is used to set up the background negative control. 48-hours after infection lentivirus expression was read by a Gallios Flow Cytometer (Beckman Coulter) and Kalluza Software. copGFP marker expression was measured by qRT-PCR as positive control for lentiviral expression and miR-326 levels were measured by qRT-PCR to ensure the miRNA modulation by LVs.

### PROLIFERATION STUDIES

### BrdU Cell Proliferation Assay

This assay was carried out in Caco-2 and MC-38 cells at 48h and 72h post miR-326 modulation, respectively, as specified above. The following day, cells were harvested and 1,5×10⁴ Caco-2 and 1x10⁴ MC-38 cells were seeded on p96-well by triplicate. Furthermore, a triplicate of non-transfected cells was used as background control. Blank determination was performed by adding DMEM without cells. 24 hours later, 10 µL of 1/100 BrdU Labeling Reagent diluted with DMEM was added and incubated for 24h at 37°C. Background wells had not BrdU Labeling Reagent. BrdU processing was carried out following the manufacturer's instructions (#11647229001, Roche): 200 µL of FixDenant for 30 minutes at room temperature; after removing this, 100 µL of 1/100 Anti-BrdU-POD Working Solution was diluted with Antibody dilution Solution in darkness during 90 minutes at room temperature. Then, 3 washes of 250 µL 1/10 Washing Buffer in distilled water were made. Finally, 100 µL of Substrate Solution was added to each sample for quantification in an absorbance spectrophotometer at 370 nm (Multiskan GO). The optimal time for the substrate reaction was 10 and 5 minutes in Caco-2 and MC-38 respectively. Blank provides information about the unspecific binding of BrdU and anti-BrdU-POD conjugate to the plate. Background control provides information about the unspecific binding of the anti-BrdU-POD conjugate to the cells in the absence of BrdU.

### Cell Cycle Distribution by Propidium Iodide Assay

miR-326 expression was modulated in Caco-2 cells with Pre-miRs and Anti-miRs. After 72 hours, cells were harvested, washed with 1X PBS, and fixed in cold 70% ethanol at -20°C in FACS tubes for 48h. Following, cells were washed, resuspended in 500 µL of 1X PBS and treated with 0.5 µg of RNase A (#12091021, Thermo Fisher), this ensures only DNA, not RNA, is stained. 5 µg of Propidium Iodide (#P1304MP, ThermoFisher) was added to each FACS tube and cell cycle distribution was determined with a 4-laser Attune NxT acoustic cytometer (ThermoFisher Scientific, Waltham, MA, USA). The percentage of cells in G0/G1, S, and G2/M phases was determined using FlowJOv10 program.

### CELL ADHESION ASSAY TO FIBRONECTIN

This experiment was carried out in Caco-2 cells after modulation of miR-326 as specified above. p24-well plates were treated with Fibronectin at 10 µg/ml for 1 hour at 37°C. Fibronectin matrix was washed 3 times with 1X PBS and 2×10⁵ cells/well were seeded on fibronectin matrix wells. One hour later, the medium was removed and washed twice with 1X PBS. Finally, attached cells to the fibronectin matrix were detached with 0.25% Trypsin-1X EDTA, stained with a vital dye, and counted with LUNA^{™} Automated Cell Counter (Logos Biosystems). This assay was carried out in triplicate for each condition.

### MIR-326 TARGETS IDENTIFICATION & VALIDATION

### Targets Prediction

A bioinformatic target prediction study was performed in order to determine predicted and validated miR-326 targets involved in the development and progression of CRC. In this bioinformatic analysis, several online databases were consulted, TargetScan, miRbase, and miRtarbase. Based on this analysis, we identified two miR-326 predicted and non-validated target family genes. The integrin α (ITGA) and the chemokines (CXC) genes. The gene encoding the α-5 β-1 integrin (ITGA5) acts as a fibronectin receptor and is correlated with proliferation, migration and poor overall survival in CRC (Lu et al. 2019a). Moreover, CXCR4 overexpression has been identified as a negative prognostic factor and distant dissemination in CRC (Ottaiano et al. 2021a). Based on this evidence, we hypothesized that these genes could be regulated by miR-326 in our *in vitro* system.

### Targets identification: Luciferase Reporter Assays

Integrin alpha 5 (ITGA5) (NM_002205; #SC212067, Origene) and CXCR4 (NM_001008540; #SC207204) 3' UTR sequence were cloned separately, into downstream of the Firefly luciferase gene, in the Multiple Cloning Site pMiRTarget vector (*Figure 2*). The empty pMiRTarget vector was used as the control of the assay. DH5α Competent Cells (#18265017, Thermo Fisher) were used to amplify these three vectors according to the manufacturer's instructions. DNA Plasmids were obtained after purification using EndoFree Mini-Prep and Maxi-prep Plasmid Kits (#12123 #12162, Qiagen). DNA of the three pMiRTarget Vectors was sequenced with the sequencing primers to confirm the clones (Forward 5'AGAAGCTGCGCGGTGGTGTTGTG3' and Reverse 5'CTGGAGGATCATCCAGCCGGCGT3'). pRL-SV40 Vector (Promoter-Driven Control Renilla Luciferase Vector, #E6911, Promega) was used for normalization in luciferase assays.

For luciferase assays, Caco-2 cells were cultured in p24-well plates and transfection was performed at 70-80% of confluence. 500 ng of pMiRTarget Vector (containing a firefly luciferase gene, ITGA5 3'UTR or CXCR4 3'UTR), 0.5 ng of pRL-SV40 Vector (containing a Renilla luciferase gene) and Pre-miR-326 or Pre-miR-Scrambled with a final concentration of 0.25 nM, were simultaneously transfected per well using 2.5 µL of lipofectamine. Each transfection condition was carried out in quadrupled. For control, cells were co-transfected with pMiRTarget Empty Vector, pRL-SV40 Vector and Pre-miR-326 or Pre-miR-Scrambled. Firefly and Renilla luciferase activity were measured after 48h of transfection using Dual-Luciferase Reporter System (#E1910, Promega) following the manufacturer's instructions, in an Orion LB 965 Microplate Luminometer (Berthold Technologies). Luciferase activity was normalized using Renilla values for each sample.

### GENES & MIRNAS EXPRESSION & LOCATION STUDIES

### RNA isolation

### Total RNA extraction from cell culture

Total RNA from cell cultures was extracted with guanidine thiocyanate-phenol-chloroform method using TriPure Isolation Reagent solution (#11667165001, Roche), following the manufacturer's instructions: mechanical lysis, phenol/chloroform extraction, and subsequent alcohols precipitation.

Besides, to maximize the efficiency of RNA isolation in murine macrophages, glycogen (#10814010, Invitrogen) was added to Tripure Isolation Reagent [20 µl/ml], and linear acrylamide (#AM9520, Invitrogen) was used in the alcohol precipitation step (3µl/tube).

### Total RNA extraction from FFPE tissues

Punches obtained from paraffin-embedded tumour biopsies were crushed with a scalpel blade to favour further tissue digestion. Total RNA enriched in miRNAs was extracted with miRNeasy FFPE kit (# 217504, Qiagen), following the manufacturer's indications using enrichment columns purification and samples were stored at -80°C before qRT-PCRs.

### RNA Quantification and Integrity study by Electrophoresis

Concentration and purity of the obtained RNA were estimated with a Nanodrop 2000device (Thermo Fisher) and considering the A260/280 and A260/230 ratios. RNA integrity was checked by electrophoresis in agarose gels loading a solution mix composed of 1 µl of total RNA, 1 µl of DNA-Dye NonTox (A9555, PanReac AppliChem) and 4 µl of nuclease-free water. After electrophoresis, RNA bands were visualized under a UV-transilluminator, ChemiDoc MP Imaging System (BioRad). Finally, RNA was stored at -80°C until its use.

### Quantitative Real-time Polymerase Chain Reaction (qRT-PCR)

### qRT-PCR for Messenger Expression

Reverse transcription (RT) to obtain cDNA from the isolated RNA was performed using a Transcriptor First Strand cDNA Synthesis Kit (#04897030001, Roche). Following the manufacturer's instructions, 2 µg of RNA from each sample were used for the reaction. Once RT finished, 1 µl of the obtained cDNA was used as a template for the qRT-PCR, together with SYBR Green I Master Mix (#04887352001, Roche). Amplification was carried out in a LightCycler 480 thermal cycler (Roche) with the following temperature protocol: 40 cycles at 95°C for 10s, 60°C for 20s, and 72°C for 20s. All reactions were performed by triplicate for each sample, and 28S ribosomal gene was selected as an internal control (housekeeping gene) for data normalization. Besides, cDNA samples were diluted 1/100 with sterile nuclease-free water to 28S gene amplification, and no diluted for the rest of the studied genes. Results were expressed as crossing points (CTs) with the 2^{nd} derivative method (LightCycler 480 Software 1.5) and data analysis for gene expression values was presented as ΔCTs obtained as follows: ΔCT = Gene CT - housekeeping CT, and folds calculated using the 2^{-ΔΔCT} formula. Specific primers (oligonucleotides) employed are detailed in *Table* . Primers were synthetized by TIB Molbiol in a 100 µM stock concentration and diluted in a use concentration of 1:10 in nuclease-free water.

**Table 2. Oligonucleotides used for messenger expression by qRT-PCR. Product specifications and Forward and Reverse sequences are detailed.**

| **Gene primers (mRNA)** | | | |
|---|---|---|---|
| **Gene** | **Specie** | **Forward sequence 5'-3'** | **Reverse sequence 5'-3'** |
| **28S** | Human/Mouse | CAGTACGAATACAGACCG | GGCAACAACACATCATCAG |
| **CXCR4** | Mouse | GACTGGCATAGTCGGCAATGGA | CAAAGAGGAGGTCAGCCACTGA |
| **copGFP** | Human/Mouse | CCGCATCGAGAAGTACGAGG | CTGCGGATGATCTTGTCGGT |
| **E-Cadherin** | Human | ACACCAACGATAATCCTCCGA | CATCAGCATCAGTCACTTTCAG |
| **ITGA5** | Mouse | AGCTGGATGTGTATGGGGAG | CAGCTCAGGCTGGAGAAGTT |
| **N-Cadherin** | Human | CGAATGGATGAAAGACCCATCC | GGAGCCACTGCCTTCATAGTCAA |
| **NOS2** | Mouse | AGACCTCAACAGAGCCCTCA | AAGGTGAGCTGAACGAGGAG |
| **PDL1** | Human | GGTGCCGACTACAAGCGAAT | GGTGACTGGATCCACAACCAA |
| **PDL1** | Mouse | TGCGGACTACAAGCGAATCACG | CTCAGCTTCTGGATAACCCTCG |
| **Snail** | Human | CATCCTTCTCACTGCCATG | GTCTTCATCAAAGTCCTGTGG |
| **TWIST1** | Human/Mouse | GCCAGGTACATCGACTTCCTCT | TCCATCCTCCAGACCGAGAAGG |
| **TWIST2** | Human/Mouse | GCAAGATCCAGACGCTCAAGCT | ACACGGAGAAGGCGTAGCTGAG |
| **YM1** | Mouse | AAGAACACTGAGCTAAAAACTCTCCT | GAGACCATGGCACTGAACG |
| **ZEB1** | Human/Mouse | TACAGAACCCAACTTGAACGTCACA | GATTACACCCAGACTGCGTCACA |

### qRT-PCR for miRNA Expression

cDNA for miRNAs expression study was generated using the miRCURY LNA Universal RT microRNA, Polyadenylation, and cDNA Synthesis Kit II (#339340, EXIQON). Drown from the manufacturer's recommendations, 100-200 ng of isolated RNA from cells or paraffin-embedded tissue were used. As a control of cDNA synthesis efficiency, an external RNA (cel-miR-39) was added and further amplified. After, 4 µl of the obtained cDNA, 1/11 diluted in nuclease-free water, were added as a template for the qRT-PCR in each reaction sample, with 6 µl of the master mix including 5 µl of SYBR Green (#339347, Qiagen) and 1 µl of specific LNA probes for each selected miRNA. Amplification was performed in a Light Cycler 480 thermal cycler (Roche) following the next temperature protocol: enzyme activation at 95°C for 10 min, 45 cycles at 95°C for 15s, and finishing with 60°C for 60s. All reactions were carried out in triplicate using a LightCycler 480 instrument and Ct values were calculated using a 2^{nd} derivative method. miRNA expression values are presented as ΔCTs obtained as follows: ΔCT = miRNA CT - housekeeping CT, using the 2^{-ΔCT} formula, or folds, using the 2^{-ΔΔCT} formula. Normfinder and Bestkeeper software were used to determine the most stable housekeeping miRNAs. miR-191-5p and miR-103a-3p were finally selected as housekeeping for data normalization in case of colorectal cancer cells and murine macrophages. The mean of 5S rRNA Cq and RNU6B Cq as housekeeping was used for normalization in tissue samples from patients' biopsies. All LNA miRNAs probes used in this work were obtained from Qiagen and used at a final concentration of 10 µM (Error! Reference source not found.).

**Table 3, Oligonucleotides (LNA miRNAs probes) used for miRNA \ expression by qRT-PCR. Oligonucleotides (LNA miRNAs probes) ! used for miRNA expression by qRT-PCR. Product specifications and reference numbers are detailed.**

| **miRNas primers** | | |
|---|---|---|
| **miRNA** | **LNA name** | **Reference number** |
| **Cel39** | cel-miR-39-3p | YP00203952 |
| **miR-103** | hsa-miR-103a-3p | YP00204063 |
| **miR-191** | hsa-miR-191-5p | YP00204306 |
| **miR-326** | hsa-miR-326 | YP00204512 |
| **5S** | 5S rRNA | YP00203906 |
| **U6B** | U6 snRNA | YP00203907 |

### miRNAs in-situ hybridization (ISH)

In situ hybridization uses a complementary RNA probe to localize a specific RNA sequence, in this case, a miRNA in the tissue. To start the protocol, deparaffinized and rehydrated colorectal sections were treated with Proteinase K (#158918, Qiagen) (3 µL/ml in PBS 1X diluted in DEPC water) for 15 minutes at 37°C to digest the tissue and allow the probe to penetrate in the cells. Then, they were washed with diethylpyrocarbonate-treated (DEPC-treated) water for 5 min at room temperature to remove the exceeded product. After that, 25 µl of the specific probe solution was added over tissue sections to detect the studied miRNA (hsa-miR-326), or the internal control (housekeeping) (hsammurnoU6), diluted in Formamide-free microRNA ISH Buffer (#90-012, Exiqon) and DEPC-treated water (probe solution: 1 µl of the probe (studied miRNA or housekeeping)/250 µl of DEPC-treated water/250 µl of Formamide-free microRNA ISH Buffer). Slides were covered and sealed with Fixogum rubber cement (#290110001, Marabu) to avoid evaporation, and incubated overnight at 37°C in a humidified chamber. The next day, samples were washed in 5X SSC for 5 min at 60°C and incubated in 1X Blocking Reagent Solution (#11096176001, Roche) for 5 min at room temperature. Later, 200 µl of DIG-AP antibody (#11093274910, Roche) (1:800) was added to each slide diluted in 1X Blocking Reagent Solution with 10% of sheep serum (#S2263, Sigma-Aldrich) and incubated for 1h at room temperature. After two washes in PBS Tween (PBST) 0.1% v/v for 5 min each one, slides were treated with 150 µl of chromogen solution: 200 µl of NBT-BICP Stock Solution (#11681451001, Roche) diluted in 10 ml of 0.1 M Tris HCl/NaCl pH 9.5, for 2h at 37°C, again in a humidified chamber. Over this time, tissue sections were washed again in PBST for 5 min at room temperature two times, and two more in DEPC-treated water. Finally, nuclei in the tissue sections were stained by incubation with Nuclear Fast Red solution (#N3020, Sigma-Aldrich) for 1h at room temperature. Stain excess was removed under running water before dehydration in the xylene and alcohol solutions sequence, but starting in alcohol and finishing in xylene, and mounting with FLUKA Eukitt mounting medium (#03989, Fluka) to be analysed and evaluated under the microscope. A pathologist analysed the CRC biopsies determining and quantifying the expression of miR-326 in the different cell compartments of the slide. A hierarchy was established on which compartments had the highest expression of miR-326 in each tissue.

### PROTEIN STUDIES

### Protein Expression in cells

### Protein Extraction from cell cultures

Protein extraction was performed by scraping the p60/p6 culture plates with 600/300 µL RIPA Buffer (#R0278, Sigma-Aldrich), supplemented with protease inhibitors: 1mM DTT (#43815, Sigma), 10 µg/ml Leupeptin (#L9783, Sigma), 10 µg/ml Aprotinin (#A1153, Sigma), 1mM Pefabloc (#76307, Fluka) and a cocktail of phosphatase inhibitors (#524625, Millipore). The cell lysate was collected and kept in constant agitation on a shaker rotatory mixer for 20 minutes at 4°C. Finally, it was centrifuged for 10 minutes at 15000 rpm at 4°C to precipitate the insoluble residues. Supernatants were collected and stored at - 80°C for experimental approaches.

### Immunoblot (Western Blot Analysis)

Total protein concentration in cell lysate was estimated by Bradford colorimetric technique. A standard curve was made with serial dilutions of BSA (#10711454001, Sigma-Aldrich), and 5 µL of protein lysate was used for quantification at 595 O.D. in an absorbance spectrophotometer (Multiskan GO, Thermo Fisher). 50 µg of total proteins were electrophoretically separated into SDS-polyacrylamide gels (Biorad) under denaturing conditions and transferred to nitrocellulose membranes (#iB23001, Invitrogen), in an iBlot^{™} 2 Gel Transfer Device (Invitrogen). Nitrocellulose membranes were incubated in a 1X TBS blocking solution + 5% BSA + 0.1% Tween (#142312, Panreac Química S.A.), for 1h at room temperature. After this, the membranes were incubated overnight at 4°C with the corresponding dilutions of primary antibodies in 1X TBS + 5% BSA + 0.1% Tween. Primary antibodies used: anti-p27 (27 KDa), 1:500 (610241, BD Biosciences); anti-ITGA5 (115 KDa), 1:200 (sc-376199, Santa Cruz Biotechnologies); anti-SNAI1 (29 KDa) 1:500 (C15D3, Cell Signaling Technology); anti-CXCR4 (40KDa) 1:250 (ab124824, Abeam); anti-β-actin (42KDa) 1:1000 (Sigma-Aldrich). The following day, three 5-minute washes were performed with 1X TBS-0.1% Tween and incubated with the corresponding secondary fluorescent antibody for 1 hour at room temperature with agitation (Ll-COR Biosciences, 926-68070/926-68071). Finally, three 5-minute washes were performed with 1X TBS-0.1% Tween, before displaying the result with the fluorescence reader (Odyssey). Image Analysis and Quantitation for Western Blotting were carried out using Scion Image Program.

### Protein Localization in Tissues

### Tissue Processing

Formalin-fixed and paraffin-embedded (FFPE) tissues were sectioned into 3-4 µm thick slices using a microtome (Microm HM 325, Thermo Scientific) and placed into polysine covered microscopic-slides (#K802021-2, Dako). To perform histological staining, tissues were deparaffinized in an oven for 1 h at 60°C, and sequentially incubate for 10 min at room temperature in xylene, 100% alcohol, 95% alcohol, 70% alcohol solutions, and finally, in water for 20 min, to rehydrate the tissue. After the subsequent stain, the slides were observed by means of a phase-contrast optical microscope and evaluated by a specialized pathologist. Tissue images were acquired using a digital camera incorporated into a Nikon Eclipse TE2000-U microscope.

### Immunohistochemistry

Immunohistochemistry was performed to visualize protein expression content and localization in tissue samples through the detection of specific epitope-antibody interactions, also labelled. 3µm sections of FFPE tumour biopsies were rehydrated and heat-treated using a pressure cooker to unmask the antigenic epitopes. Antigen retrieval solution and boiling time suitable for each antibody are specified in Error! Reference source not found.. Then, slides were tempered for 20 min immersed in the preferred retrieval solution and washed for 5 min in 1X PBS.

From this moment on, the whole procedure was carried out inside of a wet chamber. Tissueendogenous peroxidase activity was blocked to avoid non-specific signals during immune-detection activity by incubating slides in the Peroxidase Blocking Solution (#S202386-2, Dako) for 30 minutes at room temperature. To continue, they were washed three times in the 1X PBS-0.05% Tween. After that, tissues were treated with 1X TBS with 1% v/v bovine serum albumin solution (BSA, #A7906, Sigma-Aldrich) for 30 min at room temperature to avoid non-specific antibody binding. Samples were incubated with the primary antibody diluted following the specification of the data sheet (Table 4), overnight at 4°C in a humidified chamber. The next day, slides were washed three times in 1X PBST for 5 min before incubating them with the secondary antibody, HRP-conjugated, for 30 min.

Then, after three more washes of 5 min in 1X PBST, the signal was developed using a detection kit containing DAB substrate as chromogen (#GV82511-2, Dako). The reaction was stopped when tissues appear coloured after applying water. To finalize, a counterstain with Harris' Hematoxylin Solution (#1.09253.0500, Merk), 2 min at room temperature, was performed in the samples to distinguish cellular compartments in the stained tissues. Tissues were dehydrated incubating in the xylene and alcohol solutions sequence, but starting in alcohol and finishing in xylene, and mounted with DEPEX medium (#3808600E, Laica Biosystems) to be analysed and evaluated under the microscope by a pathologist specialized in CRC diseases. Immunostaining of CXCR4, ITGA5, and immune infiltrate molecules (CD56, CD68, CD8, CD25, CD163, CD11c, Myeloperoxidase, Granzyme B and Perforin) were evaluated. The evaluation of the immunostainings was carried out in two compartments (intratumoral and peritumoral area). The intratumoral evaluation was made in the central area of the tumour, excluding as far as possible the superficial area (1/3). The peritumoral evaluation was made on the deep border of infiltration. Six high magnification fields (40x objective) were chosen randomly in each compartment and positive cells were counted. The result is a SCORE calculated by the average of the six 40x fields. Furthermore, the quantification of each marker was evaluated following different methodologies:
- CXCR4 was evaluated in the intratumoral and peritumoral areas as was mentioned before. CXCR4 staining was analysed by combining two variables: extension and intensity of the stain and a SCORE was acquired from these two variables.
- ITGA5 was evaluated in the intratumoral and peritumoral areas of the CRC tissues.
- CD56 was evaluated in the membrane of neuroectoderm-derived cells and NK cell-derived lymphomas in intratumoral and peritumoral areas of the CRC tissue.
- CD68 was evaluated in the cytoplasm and the membrane of monocytes and M1 macrophages in intratumoral and peritumoral areas of the CRC tissue.
- CD8 was evaluated in the membrane of cytotoxic T-cell lymphocytes in intratumoral and peritumoral areas of the CRC tissue.
- CD25 was evaluated in the cytoplasm and the membrane of T cells and mastocytes in intratumoral and peritumoral areas of the CRC tissue.
- CD163 was evaluated in the cytoplasm and the membrane of activated or anti-inflammatory M2 macrophages in intratumoral and peritumoral areas of the CRC tissue.
- CD11c was evaluated in the cytoplasm of dendritic cells in intratumoral and peritumoral areas of the CRC tissue.
- Myeloperoxidase was evaluated in the cytoplasm of granulocytes and monocytes in intratumoral and peritumoral areas of the CRC tissue.
- Granzyme B was evaluated in the cytoplasm of cytotoxic lymphocytes (CTL or NK cells) in intratumoral and peritumoral areas of the CRC tissue.
- Perforin was evaluated in the perinuclear cytoplasm of cytotoxic lymphocytes (CD4+, CD8+ or NK cells) in intratumoral and peritumoral areas of the CRC tissue.

**Table 4. Primary antibody used for immunohistochemistry staining. Product specification, procedure followed, ; and IHC-related product used for each one.**

| **Immunohistochemistry Staining** | | | | | | |
|---|---|---|---|---|---|---|
| **Primary antibody** | **Source** | **Reference** | **Dilution** | **Antigen Retrieval Solution** | **Antigen Retrieval Time** | **Secondary Antibody** |
| **CXCR4** | Rabbit | Ab124824 Abcam | 1/100 | Citrate, pH9 | 5 min | Envision Dako, K4065 |
| **ITGA5** | Mouse | Ab150361 Abcam | 1/100 | Citrate, pH6 | 5 min | Envision Dako, K4065 |
| **CD56** | Rabbit | Ab220360 Abcam | 1/500 | Citrate, pH6 | 10 min | Envision Dako, K4065 |
| **CD68** | Rabbit | Ab125212 Abcam | 1/100 | Citrate, pH6 | 10 min | Envision Dako, K4065 |
| **CD8** | Rabbit | Ab93278 Abcam | 1/250 | Citrate, pH6 | 15 min | Envision Dako, K4065 |
| **CD25** | Mouse | Cell Marque 4C9 | 1/50 | Citrate, pH6 | 15 min | Envision Dako, K4065 |
| **CD163** | Mouse | Ab74604 Abcam | 1/300 | Citrate, pH9 | 15 min | Envision Dako, K4065 |
| **CD11c** | Rabbit | Ab52632 Abcam | 1/250 | Citrate, pH9 | 5 min | Envision Dako, K4065 |
| **Myeloperoxydase** | Rabbit | Thermo Fisher PA5-16672 | 1/75 | Citrate, pH6 | 20 min | Envision Dako, K4065 |
| **Granzyme B** | Rabbit | Ab4059 Abcam | 1/100 | Citrate, pH6 | 10 min | Envision Dako, K4065 |
| **Perforin** | Mouse | Ab75573 Abcam | Prediluited | Citrate, pH6 | 15 min | Envision Dako, K4065 |

### STATISTICAL ANALYSIS

The normal distribution of variables was assessed with the Shapiro-Wilk test. For normal distributed data, t-test and ANOVA with post hoc Bonferroni correction for multiple comparisons were used after assessing homogeneity of variances with the Levene test. For group comparison of non-normal distributed data, the Kruskal-Walli's test was performed. Intergroup differences of non-normal data were assessed with post hoc Mann-Whitney U-tests. Paired samples comparison to confront tumoral and non-tumoral biopsies followed t-test. Overall survival (OS) curves were calculated by the Kaplan-Meier method and the log-rank test was used to determine the difference in OS rates between the two groups. The median value of miR-326 expression in all samples was chosen as the cut-off point for separating low- and high-level expression. The correlation between miR-326 and the inflammatory infiltrate, ITGA5 and CXCR4 is carried out through linear regression with 95% of confidence of the number of positive cells and miR-326 expression is expressed as 2^{-ΔCT}. Statistical Package for the Social Sciences (SPSS) software version 19.0. was used for the analysis.

### Results

For this invention we have studied the role of miR-326 in the development and progression of CRC and we unveiled mechanisms involved using CRC patients' biopsies and modulating miR-326 in colon cancer and inflammatory cells.

### MIR-326 EXPRESSION IN PRIMARY TUMOUR BIOPSIES OF CRC PATIENTS

### MIR-326 EXPRESSION IS DECREASED IN TUMOUR TISSUES OF CRC PATIENTS

First, we analysed miR-326 expression levels in CRC tumour biopsies and compare them to the normal mucosa adjacent tissue of 27 CRC patients. After RNA extraction from FFPE samples, hsa-miR-326 expression was determined using qRT-PCR. Mean of miR-103 and miR-191 Cq values were used as normalizer.

As can be observed in *Figure 3**,* lower expression levels of miR-326 are shown in tumor compared with adjacent non-tumoral mucosa. A relevant statistical difference was found between tumoral and non-tumoral tissue, indicating that CRC associates with a decrease in miR-326 expression.

### MIR-326 EXPRESSION IS ASSOCIATED WITH TUMOUR SITE, STROMA PERCENTAGE AND IMMUNE INFILTRATE

As mentioned before, we analyzed the association between miR-326 expression according to different histopathological features available in a 192-patients cohort as presented in Table 1. miR-326 levels were determined by qRT-PCR in tumour biopsies. Statistical analysis demonstrates that miR-326 is upregulated in left-side patients' colorectal cancers (LCRC), compared to right-side colorectal tumours (RCRC), as can be observed in *Figure 4A**.* In addition, Figure 48 shows that higher expression levels of miR-326 were found in CRC biopsies with reduced stroma compared to the patients with pronounced stroma, which have lower miR-326 expression. Furthermore, high levels of miR-326 are associated with an increased inflammatory infiltrate compared to those biopsies exhibiting low infiltrate (*Figure 4C*)*.*

These results demonstrate that miR-326 expression is associated with different histopathological features of CRC tumors, such as tumour location, stromal component, and presence of inflammatory infiltrate, all related differently to disease progression and prognosis.

### MIR-326 EXPRESSION IS ASSOCIATED WITH CRC PROGRESSION: ASSOCIATION TO TUMOUR STAGING AND INVASION

Furthermore, we studied the association between miR-326 expression in tumour biopsies with tumour staging and invasion in our CRC patient's cohort. The results shown in *Figure 5A* demonstrate that miR-326 expression is associated with the tumour stage and exhibit a significant statistical difference among low TNM (tumour-node-metastasis TNM system) stages (I-II) and high TNM stages (III-IV). Increased levels of miR-326 are observed in earlier stages of the disease (I-II) compared to late stages (III-IV). Consistently to the tumour stage, miR-326 expression is associated with tumour invasiveness grade (T1-T4) as can be observed in Figure 58. miR-326 is upregulated in less invasive tumour grades (Tis (tumour in situ)-T1-T2) compared to late stages of invasiveness (T3-T4). Moreover, *Figure 5C* shows that miR-326 expression is also related to the lymph node's affectation. miR-326 levels are upregulated in tumors with lymph nodes non-affected.

### MIR-326 EXPRESSION IS ASSOCIATED WITH CRC PROGNOSIS

Patient prognosis is a key clinical parameter for patient management. Therefore, we studied by qRT-PCR whether miR-326 expression in primary tumour biopsies could also associate with overall survival (OS) in our cohort of patients. As can be observed in *Figure 6**,* expression levels of miR-326 were associated with the patient's overall survival. High levels of miR-326 were significantly associated with better patients' OS. This outcome agrees with the ones described in Figures 4 and 5 since high levels of miR-326 correlate with LCRC, early tumour stages, and pronounced immune infiltrate, parameters related to a good prognosis of the disease. Conversely, lower levels of miR-326 correlate with higher tumour stroma percentage and advanced tumour stages. All these findings suggest miR-326 could be used as a prognostic biomarker.

### MIR-326 LOCALIZATION IN TUMOUR CELL COMPARTMENTS

The results described above demonstrated that miR-326 expression is associated with progression and prognosis in CRC. In order to further study the role of miR-326 in this context, the next step in our invention was to determine in which cellular compartments miR-326 was located. For this purpose, we carried out in situ hybridizations of miR-326 in 40 biopsies of our cohort ofCRC patients. A pathologist specialized in CRC disease evaluated the precise localization of the microRNA. *Figure 7* shows representative images of miR-326 localization in different cellular compartments of the tumour biopsies. This evaluation revealed that the microRNA was expressed in epithelial tumour cells, fibroblasts, inflammatory cells and endothelium. Furthermore, we quantified the number of biopsies in which miR-326 was located in each cell compartment. This quantification revealed that miR-326 expression is mainly localised in tumour epithelial and infiltrating inflammatory cells.

### CHARACTERISATION OF THE INFLAMMATORY INFILTRATE IN CRC BIOPSIES

Since our results demonstrated an association between miR-326 expression and the presence of inflammatory infiltrate in the tumour biopsies of CRC and after demonstrating by in-situ hybridization that this miRNA is mainly localized in the inflammatory cells, we wanted further to characterise the inflammatory component of the CRC biopsies. For this purpose, we carried out a characterization of the inflammatory infiltrate of 40 primary tumours by immunohistochemistry of different markers of inflammatory cell populations. CD8 recognise cytotoxic T-cell lymphocytes, CD25 binds to T cells and mastocytes, CD68 recognise monocytes and M1 macrophages, CD56 identify NK cells, CD163 binds to M2 macrophages, CD11c recognise dendritic cells, Perforin and Granzyme B identify CTL and NK cells and Myeloperoxidase identify granulocytes and monocytes. A specialized pathologist examined and quantified the immunostainings. Representative images of these markers are shown (*Figure 8*)*.*

Moreover, miR-326 expression levels were determined by qRT-PCR in each sample. A correlation analysis between miR-326 levels and staining quantification of each biopsy was performed. There were no statistical differences between miR-326 expression and CD8, CD25, CD163, CD11c, Perforin, Granzyme B and Myeloperoxidase immunostaining. However, we found a positive correlation between the number of CD68, and CD56 positive cells with miR-326 expression levels in CRC biopsies (*Figure* 9). As we previously mentioned, CD68 is highly expressed in M1 macrophages and CD56 is the archetypal phenotypic marker of NK cells. All these results shed light on the correlation between miR-326 and the immune infiltrate in the 192 cohort of patients: high levels of miR-326 correlate with immune infiltrate and, more specifically, with macrophages and NK cells presence in colorectal cancer biopsies.

### MIR-326 EXPRESSION IN TUMORAL AND NON-TUMORAL COLON CELL LINES

Before starting with the *in vitro* studies, we analysed the basal levels of miR-326 in different colon carcinoma cell lines in order to select an appropriate one for our study. miR-326 basal levels were determined using qRT-PCR in 3 different epithelial cell lines coming from colon adenocarcinoma (Caco-2, HT-29 and RKO), in a cell line derived from lymph nodes of CRC (SW620), and an epithelial non-tumoral colon cell line (CCD841).

As can be observed in *Figure 10**,* Caco-2 presented the highest expression levels of miR-326. Significant differences are observed between Caco-2 cells and the rest of the cell lines. miR-326 expression in HT-29 cells was 3 times lower than in Caco-2 cells. SW620 and RKO cell lines had lower expression of miR-326 than CCD841.

Since we had previously established that the miR-326 expression along the tumor biopsies decrease and we wanted to study the role of this miRNA in the disease, we chose Caco-2 cells to unveiling the role of miR-326 in the progression of CRC overexpressing and reducing its levels in this colorectal cancer cell line.

### MODULATION OF MIR-326 IN CACO-2 CELLS BY TRANSIENT TRANSFECTION

To characterize the role of miR-326 in colon cancer progression, we carried out our experiments modulating miR-326 expression in Caco-2 cells. This modulation was performed by pre-miR-326, pre-miR-Scrambled, anti-miR-326 and anti-miR-Scrambled transfection in Caco-2 cells, as we explained in the Material and Methods section. To verify the miR-326 modulation efficiency, hsa-miR-326 was amplified using qRT-PCR in modulated Caco-2 cell cultures.

As shown in *Figure 11A**,* an efficient overexpression of miR-326 was observed in the pre-miR-326 condition compared to pre-miR-Scrambled. miR-326 is 30-folds increased comparing pre-miR-326 with pre-miR-Scrambled at 24 hours and 20-folds increased at 48 hours post-transfection. On the other hand, Figure 17B shows the reduction of miR-326 expression in cells transfected with Anti-miR-326 compared with Anti-miR-Scrambled. The reduction of the miRNA is 10 folds-decreased comparing Anti-miR-326 and Anti-miR-Scrambled at both times points post-transfection. These results indicate that we can efficiently modulate the expression of miR-326 in Caco-2 cells, making suitable this cell system for the characterization of miR-326 role in CRC.

### MIR-326 OVEREXPRESSION DECREASES PROLIFERATION IN CACO-2 CELLS

Proliferation is an important process underling cancer development and progression. To determine whether miR-326 is involved in Caco-2 cell proliferation, we performed a BrdU proliferation assay in miR-326 modulated cells. BrdU is incorporated into the newly synthesized DNA of replicating cells and absorbance values correlate to the number of proliferating cells.

A statistically significant decrease in the percentage of Caco-2 proliferation rate when miR-326 is overexpressed can be observed (*Figure 12*)*.* miR-326 inhibition did not show differences in the proliferation process. These results demonstrate that miR-326 is involved in the Caco-2 cell proliferation process and overexpression of this miRNA leads to a reduction in the proliferation rate.

### 3.2 MIR-326 AFFECTS CELL CYCLE IN CACO-2 CELLS: ARREST ON S-PHASE

As was described in the introduction, uncontrolled cell proliferation is caused by altered expression and/or activity of different proteins implied in the cell cycle process. Dysregulated expression of CDKs, CDK inhibitors or checkpoint regulator proteins carry to an altered proliferation process.

As we have reported above, miR-326 is involved in the cell proliferation of Caco-2 cells and overexpression of this miRNA decreases cell proliferation. For this reason, we studied the involvement of miR-326 in the cell cycle distribution of Caco-2 cells. These studies were carried out in miR-326 modulated Caco-2 cells by staining with propidium iodide and following analysis by flow cytometry.

As can be observed in *Figure 19A**,* overexpression of miR-326 significantly arrests cells in S-phase (DNA synthesis) and decreases the number of cells reaching G2 phase compared to pre-miR-Scrambled. Any change was observed in the percentage of cells in G0/G1 phase. Conversely, inhibition of miR-326 significantly reduces the percent of cells in S-phase and increases the ones in G2 phase (*Figure 19B*)*.* These results show a reduced number of cells entering in G2 when miR-326 is overexpressed, by arresting Caco-2 cells in S-phase. These results correlate with our previous findings, and these data demonstrate that miR-326 overexpression leads to a decrease in cell proliferation.

### MIR-326 MODULATES P-27 IN CACO-2 CELLS

Once we demonstrated that miR-326 overexpression reduces proliferation arresting cells in S-phase, we went further to determine the mechanism by which this miRNA was promoting the accumulation of the cells in this phase.

Since the cyclin-dependent kinase inhibitor p27 is involved in the G1-S transition, we studied p27 expression by immunoblot in miR-326 modulated Caco-2 cells. As can be observed in *14A,* overexpression of miR-326 reduces p27 protein levels and inhibition of this miRNA increases p27 expression. β-actin was used as a loading control for the experiments. The relative quantification of p27 levels is shown in *Figure 14B**.* These results strongly suggest that miR-326 regulates p27 expression and a decrease in the G1 regulator allows the cells to accumulate in S-phase without a pertinent control.

### 3.4 MIR-326 REGULATES EPITHELIAL-MESENCHYMAL TRANSITION MARKERS IN CACO-2 CELLS

As we have previously described, lower miR-326 expression is associated with CRC progression and abundant tumour stroma in CRC tumour biopsies. Tumour stroma formation and EMT process are related in a tumoral context. To establish whether miR-326 is involved in EMT process, epithelial genes such as the tumour suppressor E-cadherin, and mesenchymal genes, such as TWIST1/2, SNAI1, N-cadherin and ZEB1 were measured by qRT-PCR in miR-326 modulated Caco-2 cells.

As we can observe in *Figure 15**,* a higher expression of E-cadherin is shown when miR-326 is overexpressed. Inhibition of miR-326 did not show any effect in this epithelial tumour suppressor gene. On the other hand, mesenchymal gene levels including TWIST1, TWIST2 or SNAI1 decreased when miR-326 was overexpressed and conversely, they increased when miR-326 was inhibited at 24h and 48h post-modulation. ZEB1 and N-cadherin didn't show any differences modulating miR-326 in Caco-2 cells.

Taken together, these data demonstrate that overexpression of miR-326 contributes to the maintenance of the epithelial phenotype, by increasing epithelial gene expression (E-cadherin) and miR-326 inhibition leads to decreasing mesenchymal genes expression at mRNA expression (TWIST1, TWIST2 and SNAI1).

### ITGA5 IS A MIR-326 TARGET IN CACO-2 CELLS

Once we confirmed that miR-326 was involved in relevant processes for tumor progression such as proliferation, cell-cycle regulation, and EMT in Caco-2 cells, we went further into putative miR-326 targets identification. First, a bioinformatic target prediction study, using different databases available online, was performed to determine validated and predicted miR-326 targets involved in the development and progression of CRC. In this bioinformatic analysis, the integrin α family, more specifically, the gene encoding the α-5 beta-1 integrin (ITGA5) was found as a miR-326 predicted and non-validated target gene. Moreover, the bibliography described that ITGA5 was correlated with proliferation, migration and poor overall survival in CRC (Lu et al. 2019). Based on this evidence, we hypothesized that this gene could be regulated by miR-326 in our *in vitro* system.

As previously described, miRNAs negatively regulate gene expression at mRNA and protein levels by degrading their mRNA targets and/or by silencing translation. To determine whether miR-326 regulates the expression of ITGA5, miR-326 was modulated in Caco-2 cells and ITGA5 protein expression was determined by immunoblot. We can observe in *Figure 16A**,* that overexpression of miR-326 in Caco-2 cells reduces ITGA5 protein levels compared with its pre-miR-Scrambled, at 24 and 48 hours post-transfection. Moreover, inhibition of the microRNA increases ITGA5 protein expression mostly at 48 hours post-transfection. These results demonstrated that miR-326 negatively regulates ITGA5 protein expression. The relative quantification of ITGA5 levels is shown in *Figure 16B**.*

To further confirm whether ITGA5 was a direct target of miR326, ITGA5 3'-UTR was cloned into pMiRTarget 3'-UTR Vectors with firefly luciferase as a reporter and luciferase activity assays were carried out. Luciferase gene expression was driven by SV-40 promoter and ITGA5 3'-UTR was cloned after the luciferase gene. pMiRTarget Empty Vector was used as control. pMiRTarget 3'-UTR ITGA5 and pMiRTarget Empty Vector were co-transfected with both Pre-miR-326 and Pre-miR-Scrambled in Caco-2 cells. Renilla luciferase activity was used for normalization control. Data are shown as the percentage of luciferase activity reduction by Pre-miR-326 in comparison to Pre-miR-Scrambled control. This experiment demonstrated that miR-326 overexpression significantly reduced luciferase activity compared with the Scrambled condition. Moreover, miR-326 overexpression did not reduce luciferase activity when was transfected with pMiRTarget Empty Vector. Statistical significance was observed between 3'-UTR ITGA5 and 3'-UTR Empty Vector transfected with miR-326. All these data demonstrate, for the first time, that miR-326 directly regulates ITGA5 expression through recognition and binding to its 3'-UTR in Caco-2 cells (*Figure 17*).

### MIR-326 REGULATES CELL ADHESION TO FIBRONECTIN THROUGH ITGA5 IN CACO-2 CELLS

It is remarkable that ITGA5 is the main receptor for soluble fibronectin. Fibronectin plays an important role in cell adhesion, spreading, migration, differentiation and high levels of fibronectin are related to cancer progression. We studied whether miR-326 was involved in cell adhesion to fibronectin, as a tumoral matrix, through ITGA5 having relevance in tumor development and progression. For this purpose, fibronectin matrix adhesion assays were carried out in Caco-2 cells previously modulated with miR-326 and the number of cells attached to a fibronectin were quantified. As can be observed in *Figure 18**,* a significant decrease in cell adhesion was observed when miR-326 was overexpressed compared to Pre-miR-Scrambled condition. A tendency of increased adhesion when miR-326 is inhibited can be observed but no statistical significance was found.

### MIR-326 REGULATES SNAIL EXPRESSION IN CACO-2 CELLS

SNAI1 gene expression is modulated by miR-326. Protein expression of SNAI1, SNAIL protein, enhances cell detachment and leads to cell migration by changing the expression of integrins (Haraguchi et al. 2008). Accordingly with these studies and following ITGA5 as a direct target of miR-326, we analysed whether this miRNA was regulating SNAIL protein levels. miR-326 was modulated in Caco-2 cells, proteins were isolated and SNAIL expression was determined by immunoblot. As we can observe in *Figure 19A*, overexpression of miR-326 reduces SNAIL protein levels with pre-miR-Scrambled at 24 hours post-transfection and inhibition of the miRNA increases the SNAIL expression at 48 hours post-transfection. The relative quantification of SNAIL levels is shown in *Figure 19B**.*

These results are showing that miR-326-ITGA5-SNAIL axis regulate cell attachment/detachment more probably regulating proteins from the extracellular matrix such as fibronectin as receptor of ITGA5.

### CXCR4 IS A MIR-326 TARGET IN CACO-2 CELLS

The bioinformatic target prediction analysis revealed another family of genes as a predicted and non-validated target of miR-326, the chemokines family (CXC) genes. As was described in the introduction of this work, CXCR4 plays an important role in EMT and progression of CRC and represents a relevant poor prognostic indicator in the development of the disease. More recently, this molecule has been involved in resistance to treatments in CRC (Goïta et al. 2022). Considering the role of miR-326 in these processes, we studied whether CXCR4 could be a miR-326 direct target. To establish if miR-326 regulates CXCR4 expression, miR-326 was modulated in Caco-2 cells and CXCR4 protein expression was determined by immunoblot. As can be observed in *Figure 20A**,* overexpression of miR-326 in Caco-2 cells reduces CXCR4 protein expression comparing pre-miR-326 with pre-miR-Scrambled. This overexpression of the protein can be observed at 24- and 48-hours post-transfection. Inhibition of the microRNA increases the expression of CXCR4 at 48-hours post-transfection. The relative quantification of CXCR4 levels is shown in *Figure 20B**.*

These results demonstrate that miR-326 negatively regulates CXCR4 expression in Caco-2 cells, being CXCR4 a possible target of miR-326. To further confirm that CXCR4 is a miR-326 target, CXCR4 3'-UTR was cloned into pMiRTarget 3'-UTR Vectors and luciferase assays were carried out as we mentioned before in ITGA5 3'-UTR Luciferase Assays. We can observe in 1 that miR-326 overexpression significantly reduced luciferase activity compared with the Scrambled condition in the 3'-UTR CXCR4 vectors. In contrast, miR-326 overexpression did not reduce luciferase activity compared with the Scrambled when transfected with pMiRTarget Empty Vector. Statistical significance was observed between 3'-UTR CXCR4 and 3'-UTR Empty Vector transfected with miR-326. Data are shown as the percentage of luciferase activity reduction by Pre-miR-326 in comparison to Scrambled control. All these data demonstrate for the first time that CXCR4 expression is directly regulated by miR-326 through recognition and binding to its 3'UTR in Caco-2 cells.

Our results demonstrated for the first time that miR-326 directly targets CXCR4 in Caco-2 cells.

### MIR-326 CORRELATIONS WITH ITGA5 & CXCR4 IN TUMOUR PRIMARY BIOPSIES

Once we demonstrated that ITGA5 and CXCR4 are two direct targets of miR-326 in Caco-2 cells, we went further to study whether miR-326 expression could be related to these proteins' expression in colorectal cancer tumour biopsies. For this purpose, immunohistochemistry of ITGA5 and CXCR4 were performed in 40 of our CRC patients' cohort. Again, a pathologist specialized in CRC evaluated the expression of these proteins in the tumour tissues. miR-326 expression was studied in these biopsies by qRT-PCR and correlation studies between were carried out. *Figure 22A* shows a representative image of ITGA5, and an inverse correlation between miR-326 and ITGA5 expression is observed. High levels of miR-326 correlate to low levels of ITGA5. *Figure 22B* shows a representative image of CXCR4 and the pertinent correlation study between miR-326 and CXCR4. As ITGA5 correlation, an inverse correlation between miR-326 and CXCR4 expression is observed. In concordance with the experiments carried out *in vitro,* our results have demonstrated both molecules as miR-326 targets and confirm the relation between miR-326 and ITGA5 and CXCR4 also in patients' biopsies.

### SUCCESSFUL POLARIZATION OF M1[LPS + IFNγ] AND M2[IL-4] AFTER STIMULATION

Since our previous results demonstrated a correlation between miR-326 expression and macrophage presence in CRC patients' biopsies, we went further in our study, addressing the role of miR-326 in macrophage polarization. For this, we used murine macrophages naïves Mφ[Non-stimulated] and different phenotypes induced by exogenous stimuli (M1[LPS + IFNy] and M2[IL-4]).

First, we studied the expression of NOS2 and YM1, as pro-inflammatory (M1) and anti-inflammatory (M2) macrophage markers, respectively, by qRT-PCR. In order to evaluate if macrophage polarization was achieved by exogenous stimulation: [LPS + IFNy] for M1 and [IL-4] for M2, results are presented in *Figure 23**.* We can observe that NOS2 is highly expressed in the M1[LPS + IFNy] phenotype compared to Mφ[Non-stimulated] macrophages. On the contrary, the M2[IL-4] phenotype, as an anti-inflammatory macrophage, expresses higher amounts of YM1 compared to naive macrophages (Mφ[Nonstimulated]) and the pro-inflammatory phenotype (M1[LPS + IFNy]). These results demonstrated that stimulations successfully polarized macrophages towards different phenotypes, which could be relevant in the development and establishment of tumour microenvironment.

### MIR-326 EXPRESSION IN MACROPHAGES PHENOTYPES M1[LPS + IFNγ] AND M2[IL-4]

Once we verified that macrophage polarization was successfully achieved, then we studied the expression of miR-326 by qRT-PCR in the different stimulation conditions. As shown in Error! Reference source not found. the miRNA expression levels significantly change in the macrophages depending on the stimulus received. miR-326 expression is highly reduced in M1[LPS + IFNy] and increased in M2[IL4] phenotype compared to Mφ[Non-stimulated].

Pursuing our objective of investigating the role of miR-326 in the macrophage polarization process, the next step was to modulate the miRNA expression in the different polarized groups (Mφ[Non-stimulated], M1[LPS + IFNy], and M2[IL-4]). This modulation was performed by transient transfection to overexpress and inhibit the miRNA with Pre-miR-326 and Anti-miR-326 technology, respectively, and using Pre-miR-Scrambled and Anti-miR-Scrambled sequences as control groups.

*Figure 25* shows the transfection efficiency in each stimulation condition. miR-326 was significantly overexpressed in the cells treated with Pre-miR-326 compared to the control group (Pre-miR-Scrambled). On the other hand, miR-326 expression was significantly inhibited in Anti-miR-326 treated group compared to the control (Anti-miR-Scrambled). This efficient miR-326 modulation was obtained in the three different macrophage stimulations Mφ[Non-stimulated], M1[LPS + IFNy] and M2[IL4].

### EFFECT OF MIR-326 MODULATION IN MACROPHAGES POLARIZATION

Finally, we investigated the effect of miR-326 modulation in macrophages polarization. For this purpose, we analyzed NOS2 and YM1 expression, as M1 and M2 markers, in the different polarized groups (Mφ[Non-stimulated], M1[LPS + IFNy], and M2[IL-4]) in which miR-326 expression was modulated. As we can observe in *Figure 26**,* NOS2 was not affected by miR-326 modulation in Mφ[Non-stimulated] neither in M1[LPS + IFNγ] macrophages. In contrast, overexpression of miR-326 in M2[IL4] macrophages increase NOS2 gene (M1 macrophage marker). On the other hand, YM1 expression (M2 macrophage marker) was not affected by miR-326 in Mφ[Non-stimulated] and in M1[LPS + IFNγ] macrophages. In contrast, YM1 expression levels in an M2[IL4] context decreases when miR-326 is inhibited. These results show miR-326 does not regulate macrophage polarization but is involved in the regulation of the process of shifting the M2 phenotype towards the M1 phenotype.

### MIR-326 POTENTIAL TARGETS IN MACROPHAGES

As we have previously demonstrated, CXCR4 and ITGA5 are two miR-326 direct targets in Caco-2 cells and present an inverse correlation in our tumour biopsies. Moreover, we wanted to determine if these two miR-326 targets in epithelial cells could be also modulated by miR-326 in naive macrophages (Mφ[Non-stimulated] and in induced phenotypes; M1[LPS + IFNy], and M2[IL-4]). For this purpose, CXCR4 and ITGA5 expression was studied by qRT-PCR in the different macrophages experimental groups, in which miR-326 was subsequently modulated.

We can observe in *Figure* 27 that CXCR4 did not show different expression levels between miR-326 overexpression or inhibition compared to each scrambled control in the study groups. In contrast, reduced ITGA5 expression levels were observed in miR-326 overexpressed macrophages compared to Pre-miR-Scrambled in both Mφ[Non-stimulated] and M1[LPS + IFNy] groups. Macrophages treated with Anti-miR-326 did not show statistical significance compared to the control group. M2[IL4] macrophages condition did not show modulation of ITGA5 by miR-326

These findings suggest that ITGA5 could be also a direct target of miR-326 in macrophages but further analysis by means of immunoblot and luciferase assays should be done to confirm it.

### ROLE OF MIR-326 IN PD-L1 REGULATION

Several immunotherapy drugs have probe efficacy in different types of cancer and PD-L1 represents a valuable biomarker and therapeutic target in effective immunotherapy. Recently, it has been published that miR-326 attenuates immune escape and prevents metastasis in lung adenocarcinoma by targeting PD-L1.

Based on these antecedents and our results demonstrating an association between miR-326 expression and the tumour microenvironment, we decided to study the role of miR-326 in PD-L1 regulation in CRC context. For this purpose, we studied whether miR-326 modulation could be regulating PD-L1 expression in Caco-2 cells. To determine this, we studied PD-L1 expression in miR-326 modulated Caco-2 cells by qRT-PCR. As observed in *Figure 28**,* miR-326 overexpression decreases PD-L1 levels and miR-326 inhibition increases this gene expression compared to their respective negative controls (Pre-miR-Scrambled and Anti-miR-scrambled), in 48h post-transfected Caco-2 cells.

We also studied whether miR-326 modulation could be regulating PD-L1 expression in macrophages. Therefore we analysed PD-L1 mRNA levels in our macrophage experimental groups (Mφ[Nοn-stimulated], M1[LPS + IFNy], and M2[IL-4], subsequently modulated with miR-326. As can be observed in *Figure 29**,* PD-L1 expression was regulated in Mφ[Non-stimulated] modulated macrophages. Overexpression of miR-326 reduces PD-L1 expression and inhibition of the miRNA increases PD-L1 mRNA levels in this group. Moreover, an increase of PD-L1 expression in M1[LPS + IFNy] and M2[IL-4] macrophages when miR-326 was inhibited is observed. Overexpression of miR-326 in these two experimental groups did not show any differences with its control group.

All our results reveal that PD-L1 is modulated in Caco-2 epithelial colorectal cancer cells and in macrophages by miR-326. Luciferase assays should be carried out to ensure PD-L1 as a direct target of miR-326 but these data suggest that this miRNA could be a potential biomarker for patient stratification in colorectal cancer whether immunotherapy could be potentially used.

### MODULATION OF MIR-326 IN MC-38 CELLS BY TRANSIENT TRANSFECTION

Considering the results obtained in both *in vitro* experiments and human biopsies demonstrating the important role of miR-326 in CRC development and progression, we wanted to investigate the role of this miRNA *in vivo* by setting up a CRC murine model.

For this purpose and considering the role that miR-326 may play in the regulation of the immune microenvironment, we used the MC-38 immunoresponsive murine model which has a favourable response profile to immune activation. This *in vivo* model is based on subcutaneous injection of MC-38, murine colorectal cancer cells, to develop colon adenocarcinoma. In order to determine miR-326 role in this tumour model, in our study, miR-326 was overexpressed in MC-38 cells by lentiviral vectors before being injected into the mice. Because miRNAs activity is specie-specific, first, we wanted to corroborate that miR-326 was involved in biological processes related to tumour progression in MC-38 murine cells. For this purpose, miR-326 was modulated in MC-38 cells following the same transfection protocol carried on with Lipofectamine in human Caco-2 cells. To evaluate MC-38 cells' modulation efficiency, miR-326 expression was analyzed by qRT-PCR. ***Error! Reference source not found.**1A* shows a clear overexpression of miR-326 in the pre-miR-326 condition compared to Pre-miR-Scrambled at 24-h and 48 hours. Figure ***31****B* shows the reduction of miR-326 expression in cells transfected with Anti-miR-326 compared with Anti-miR-Scrambled at 24-h and 48 hours, as we expected.

These results demonstrated that we efficiently modulate miR-326 expression in MC-38 murine cells.

### MIR-326 OVEREXPRESSION DECREASES PROLIFERATION IN MC-38 CELLS

To determine whether miR-326 was involved in cell proliferation in MC-38 cells, we performed a BrdU proliferation assay in miR-326 modulated MC-38 cells. As can be observed in *Figure 32* a statistically significant decrease in proliferation rate occurs when miR-326 was overexpressed compared to Pre-miR-Scrambled. The inhibition of the miRNA did not show differences in the proliferation process. This indicates miR-326 is involved in the proliferation process of MC-38 cells, similarly than in Caco-2 cells.

### MODULATION OF MIR-326 IN MC-38 CELLS BY LENTIVIRAL PARTICLES INFECTION

Based on the results obtained using synthetic pre-miRs and anti-miRs, we decided to use the lentiviral vectors to overexpress miR-326 in MC-38 cells trying to assure long term modulation of miR-326, required for the *in vivo* animal model. For this purpose, mouse pre-microRNA (MMIR-326) and Scrambled negative control (MMIR-000) constructs were generated, amplificated and tittered at ~9 × 10⁷ IFU/ml. After determining the titters of the LVs, MC-38 cells were infected with MMIR-326 and MMIR-000 separately to calculate the multiplicity of infection of these murine CRC cells. To determine the MOI, a gradient of infection was carried out and expression levels of copGFP were analysed. As we can observe in *Figure 33A* a better infection gradient was achived in MMIR-326 than in MMIR-000 condition. We also studied miR-326 overexpression efficiency, determining its expression levels by qPCR. *Figure 33B* exhibits that miR-326 overexpression is achieved successfully compared to its negative Scrambled which maintains miR-326 basal levels in all the conditions. A MOI of 50 was chosen for MC-38 cells to ensure miR-326 modulation guaranteeing enough virus but avoiding excessive toxicity. *In vitro* experiments were ready to develop the *in vivo* model by injection of MC-38 cells in C57BL6 mice and authorisation from the Autonomous Community of Madrid (PROEX 404.2/21) was obtained to develop this model.

## Claims

1. A composition comprising macrophages and/or natural killer (NK) cells, wherein these cells have been generated or obtained by transforming, transducing or transfecting these cells with miR-326, the seed sequence of miR-326 or a precursor of miR-326, or with a vector comprising a sequence coding for miR-326, the seed sequence of miR-326 or a precursor of miR-326, wherein the transformation, transduction or transfection is capable of significantly increasing the intracellular expression of miR-326, the seed sequence of miR-326 or a precursor of miR-326, transiently or permanently, in comparison to an untreated control macrophage or NK, and optionally, with the proviso that the composition comprises macrophages, simultaneously or subsequently to the transformation, transduction or transfection culturing these cells in the presence of a pro-inflammatory medium;
for use in the treatment of cancer in a subject in need thereof.

2. The composition for use according to claim 1, wherein the composition comprises natural killer (NK) cells, and these cells have been generated or obtained by transforming, transducing or transfecting these cells with miR-326, the seed sequence of miR-326 or a precursor of miR-326, or with a vector comprising a sequence coding for miR-326, the seed sequence of miR-326 or a precursor of miR-326, wherein the transformation, transduction or transfection is capable of significantly increasing the intracellular content or expression of miR-326, the seed sequence of miR-326 or a precursor of miR-326, transiently or permanently, in comparison to an untreated control NK, wherein said transduction, transformation or transfection in NK cells provides for an increased cytotoxicity **characterized by** a significant increased expression of cytotoxicity activation marker NKG2C with respect to an untreated control NK which does not express miR-326, the seed sequence of miR-326 or a precursor of miR-326 or that expresses basal levels of these sequences.

3. The composition for use according to claim 1 or 2, wherein the vector is a viral vector such as lentivirus, adenoviruses or adenoassociated viruses, polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates), dry powder formulations, plasmids, viruses, calcium phosphate nucleotides, aptamers, peptides and other vectorial tags.

4. The composition for use according to any one of claims 1 or 3, wherein the composition comprises macrophages and simultaneously or subsequently to the transformation, transduction or transfection of the cells, these are culture in the presence of a pro-inflammatory medium to provide a pro-inflammatory M1 phenotype.

5. The composition for use according to any one of claims 1 to 4, wherein the miR-326 is of SEQ ID NO 1 or of a sequence of nitrogen bases of nucleotide units identical in at least 80%, 85%, 90%, 91%, 92%, 93%, 94%, 95%, 96%, 97%, 98%, 99% or 99.5%, or 100% to SEQ ID NO 1, wherein the precursor of miR-326 is of SEQ ID NO 2 or of a sequence of nitrogen bases of nucleotide units identical in at least 80%, 85%, 90%, 91%, 92%, 93%, 94%, 95%, 96%, 97%, 98%, 99% or 99.5%, or 100% to SEQ ID NO 2, and wherein the seed sequence of miR-326 is of SEQ ID NO 3 or of a sequence of nitrogen bases of nucleotide units identical in at least 70%, 80%, 85%, 90%, 91%, 92%, 93%, 94%, 95%, 96%, 97%, 98%, 99% or 99.5%, or 100% to SEQ ID NO 3.

6. The composition for use according to any one of claims 1 to 5, wherein said macrophages or natural killer (NK) cells are of human origin.

7. The composition for use according to any one of claims 1 to 6, wherein said macrophages or natural killer (NK) cells are autologous or allogeneic to a human subject which is subjected to the treatment.

8. The composition for use according to any one of claims 1 to 7, wherein the disease or disorder is colorectal cancer.

9. A composition as defined in any of claims 1 to 7.

10. A composition according to claim 9, wherein the composition is a pharmaceutical composition optionally further comprising a carrier and/or adjuvant.

11. The composition of any of claims 9 or 10, for use in therapy.

12. A therapeutically effective amount of a composition comprising a miR-326, the seed sequence of miR-326 or a precursor of miR-326, or a delivery vector of miR-326, the seed sequence of miR-326 or a precursor of miR-326, such as a plasmid or vector comprising a sequence coding for miR-326, the seed sequence of miR-326 or a precursor of miR-326, wherein said composition is capable of significantly increasing the intracellular concentration of miR-326, the seed sequence of miR-326 or a precursor of miR-326 in the macrophages or natural killer (NK) cells of a subject in need thereof in comparison to an untreated control macrophage or NK;
for use in the treatment of cancer.

13. The composition for use according to claim 12, wherein said composition comprises a delivery vehicle of miR-326, or a precursor of miR-326, wherein the miR-326 is of SEQ ID NO 1 or of a sequence of nitrogen bases of nucleotide units identical in at least 80%, 85%, 90%, 91%, 92%, 93%, 94%, 95%, 96%, 97%, 98%, 99% or 99.5%, or 100% to SEQ ID NO 1, and wherein the precursor of miR-326 is of SEQ ID NO 2 or of a sequence of nitrogen bases of nucleotide units identical in at least 80%, 85%, 90%, 91%, 92%, 93%, 94%, 95%, 96%, 97%, 98%, 99% or 99.5%, or 100% to SEQ ID NO 2.

14. The composition for use according to claim 13, wherein the delivery vehicle is selected from the list consisting of viral vectors such as lentivirus, adenoviruses or adenoassociated viruses, polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates), dry powder formulations, plasmids, viruses, calcium phosphate nucleotides, aptamers, peptides and other vectorial tags.

15. The composition for use according to claim 14, wherein the delivery vehicle is a viral vector selected from the list consisting of lentivirus, adenoviruses or adenoassociated viruses.

16. The composition for use according to claim 15, wherein the delivery vehicle is a liposome or lipid carrier or nanocarrier.

17. The composition for use according to any of claims 12 to 16, wherein said composition is administered by a route selected from the list consisting of oral, rectal, vaginal, transmucosal, or intestinal administration, parenteral delivery, including intradermal, transdermal (topical), intramuscular, subcutaneous, intramedullary injections, as well as intrathecal, direct intraventricular, intravenous, intraperitoneal, or intranasal.

18. The composition for use according to any of claims 12 to 17, wherein the disease or disorder is colorectal cancer and the treatment at the effective therapeutic dose is carried out at an administration interval such that at least one symptom or feature of colorectal cancer is reduced in intensity, severity, or frequency or has delayed in onset.
